Europäisches Patentamt

European Patent Office

Office européen des brevets

(19)

(11) **EP 0 610 322 B1**

(12) **FASCICULE DE BREVET EUROPEEN**

(45) Date de publication et mention
de la délivrance du brevet:
**08.06.2005 Bulletin 2005/23**

(51) Int Cl.[7]: **C12N 15/31**, C12N 15/74,
C12N 1/21, C12Q 1/68,
C12P 21/08, A61K 39/02

(21) Numéro de dépôt: **92922373.3**

(22) Date de dépôt: **02.10.1992**

(86) Numéro de dépôt international:
**PCT/FR1992/000921**

(87) Numéro de publication internationale:
**WO 1993/007273 (15.04.1993 Gazette 1993/10)**

(54) **GENES D'HELICOBACTER PYLORI NECESSAIRES POUR LA REGULATION ET LA MATURATION DE L'UREASE ET LEUR UTILISATION**

FÜR DIE REGULATION UND REIFUNG VON UREASE NOTWENDIGE HELICOBAKTER PYLORI-GENE UND IHRE NUTZUNG

HELICOBACTER PYLORI GENES NECESSARY FOR THE REGULATION AND MATURATION OF UREASE, AND USE THEREOF

(84) Etats contractants désignés:
**AT BE CH DE DK ES FR GB GR IE IT LI LU MC NL SE**

(30) Priorité: **03.10.1991 FR 9112198**

(43) Date de publication de la demande:
**17.08.1994 Bulletin 1994/33**

(73) Titulaires:
• **INSTITUT PASTEUR**
**75724 Paris Cédex 15 (FR)**
• **INSTITUT NATIONAL DE LA SANTE ET DE LA RECHERCHE MEDICALE (INSERM)**
**75654 Paris Cédex 13 (FR)**

(72) Inventeurs:
• **LABIGNE, Agnès**
**F-91440 Bures-sur-Yvette (FR)**
• **CUSSAC, Valérie**
**F-75020 Paris (FR)**
• **FERRERO, Richard**
**F-75015 Paris (FR)**

(74) Mandataire: **Gutmann, Ernest**
**Ernest Gutmann - Yves Plasseraud S.A.**
**3, rue Chauveau-Lagarde**
**75008 Paris (FR)**

(56) Documents cités:
**EP-A- 0 367 644**        **WO-A-91/09049**

• **BULL. ACAD. NATL. M D. vol. 175, no. 6, Juin 1991, PARIS, FR pages 791 - 802 A. LABIGNE ET AL. 'Développement d'approches génétiques et moléculaires pour le diagnostic et l'étude du pouvoir pathogène de Heliobacter pylori, agent des maladies inflammatoires gastriques'**
• **BULL. ACAD. NATL. M D. vol. 175, no. 6, Juin 1991, PARIS, FR pages 791 - 802 A. LABIGNE ET AL. 'Développement d'approches génétiques et moléculaires pour le diagnostic et l'étude du pouvoir pathogène de Heliobacter pylori, agent des maladies inflammatoires gastriques'**
• **MICROB. ECOL. HEALTH DIS. (SPEC. ISSUE) vol. 4, Octobre 1991, page 139 V. CUSSAC ET AL. 'EXPRESSION OF HELIOBACTER PYLORI UREASE ACTIVITY IN ESCHERICHIA COLI HOST STRAINS'**
• **MICROB. ECOL. HEALTH DIS. (SPEC. ISSUE) vol. 4, Octobre 1991, page 136 R. FERRERO ET AL. 'CONSTRUCTION OF UREASE DEFICIENT MUTANTS OF HELIOBACTER PYLORI BY ALLELIC EXCHANGE'**
• **JOURNAL OF BACTERIOLOGY vol. 174, no. 8, Avril 1992, BALTIMORE, US pages 2466 - 2473 V. CUSSAC ET AL. 'Expression of Heliobacter pylori Urease Genes in Escherichia coli Grown under Nitrogen-Limiting Conditions'**

**Description**

Helicobacter pylori (désigné également par l'expression H. pylori) est une bactérie à gram négatif retrouvée exclusivement à ce jour à la surface de la muqueuse de l'estomac chez l'homme, et plus particulièrement autour des lésions des cratères des ulcères gastriques et duodénaux. Cette bactérie était inialement appelée Campylobacter pyloridis (Warren et al (1983) Lancet 1. 1273-1275).

Comme la plupart des bactéries, H. pylori est sensible à un milieu de pH acide mais cependant peut tolérer l'acidité en présence de taux physiologiques d'urée (Marshall et al (1990) Gastroenterol. 99: 697-702). En hydrolysant l'urée sous forme de dioxyde de carbone et d'ammoniac qui sont relargués dans le microenvironnement de la bactérie, l'uréase de H. pylori est supposée permettre la survie de la bactérie dans l'environnement acide de l'estomac. Récemment des études menées sur des modèles animaux ont fourni des éléments suggérant que l'uréase est un facteur important dans la colonisation de la muqueuse gastrique (Eaton et al (1991) Infect. Immun. 59: 2470-2475). L'uréase est également suspectée de causer des dommages soit directement, soit indirectement, à la muqueuse gastrique.

Helicobacter pylori (H. pylori) est à l'heure actuelle reconnu comme l'agent étiologique des gastrites antrales, et apparaît comme un des cofacteurs requis pour le développement des ulcères. Par ailleurs il semble que le développement de carcinomes gastriques puisse être associé à la présence de H. pylori.

Toutes les souches isolées en clinique à partir des biopsies ou de jus gastrique synthétisent une uréase très active, exposée en surface de la bactérie, qui est l'une des protéines les plus immunogènes de H. pylori. L'uréase est suspectée de jouer un rôle dans le processus pathogénique, un fait qui a été confirmé par les expérimentations réalisées sur le porc montrant que des souches faiblement productrices d'uréase obtenues par mutagénèse chimique, étaient incapables de coloniser l'estomac du porc. Ces résultats obtenus après mutagénèse chimique ne permettent cependant pas d'attribuer de façon certaine, la diminution de la production d'uréase à une inaptitude à coloniser l'estomac, d'autres gènes ayant pu être inactivés lors de la mutagénèse généralisée. Il ne s'agit donc pas de mutations contrôlables et par conséquent cette technique ne présente pas d'intérêt réel dans la conception de moyens destinés à diminuer, voire prévenir les effets néfastes de l'uréase dans le cas d'une infection par H. pylori.

Outre ce rôle dans la colonisation de l'estomac, il a été montré que l'uréase ainsi que l'ammoniac libérées pourraient avoir un effet cytotoxique direct sur les cellules épithéliales et un effet indirect en induisant une réponse inflammatoire qui serait à l'origine des lésions gastriques.

L'uréase est donc l'un des déterminants de pathogénicité les plus importants, et la construction de souches isogéniques de H. pylori inactivées spécifiquement dans les gènes responsables de l'expression de l'uréase, qu'il s'agisse des gènes de structures ou des gènes accessoires, sont de première importance pour préciser le rôle de l'uréase dans l'étape de colonisation, et pour une application à la construction de souches utilisables pour protéger les individus dans un processus de vaccination, par exemple par la construction de souches atténuées.

Jusqu'à présent les gènes de l'uréase avaient été localisés sur un fragment de 34 kb du chromosome de H. pylori et avaient été associés à une région de 4,2 kb présente dans ce fragment. Quatre gènes désignés par les termes ureA, ureB, ureC et ureD avaient été associés à cette région de 4,2 kb. Cette région permettait d'obtenir un phénotype uréase-positif lorsque l'ADN de 4,2 kb était transféré par l'intermédiaire d'un vecteur navette dans Campylobacter jejuni.

Cependant la transformation de cellules de E.coli avec l'ADN de 4,2 kb précédemment décrit ne permettait pas d'obtenir l'expression d'une activité uréasique dans E.coli.

Les inventeurs ont réussi à déterminer quels sont les éléments, tant du point de vue génétique que du point de vue des conditions de culture, nécessaires pour l'expression dans E.coli d'une activité uréasique telle qu'obtenue chez H. pylori. Ils ont à cet égard déterminé que l'expression de l'uréase chez E.coli était dépendante à la fois de l'activation du système de régulation de l'azote de E.coli et de la présence de gènes accessoires des gènes structuraux de l'uréase. Ils ont identifié et isolé plusieurs gènes que l'on désignera parfois dans la suite par l'expression "gènes accessoires" de l'uréase, qui permettent l'expression fonctionnelle de l'uréase chez E.coli et déterminent la maturation et la régulation de l'uréase chez H. pylori.

L'invention concerne donc un ensemble de cinq nouveaux gènes déterminants ou au moins susceptibles d'intervenir dans l'expression fonctionnelle de l'uréase chez H. pylori et chez E.coli, ainsi que chacun de ces gènes considérés isolément et indépendamment des autres gènes. Elle vise également cet ensemble de gènes, le cas échéant modifiés, en association avec les gènes de structure désignés par ureA, ureB, ureC et ureD de l'uréase et décrits dans la publication (Labigne et al (1991) J. Bacteriol 173: 1920-1931) .

L'invention vise par ailleurs de nouveaux moyens de détection in vitro d'une infection par H. pylori, ainsi que des compositions utilisables pour la protection contre l'infection par H. pylori.

L'invention a donc pour objet une séquence nucléotidique, caractérisée en ce qu'elle est constituée par ou en ce qu'elle comprend au moins une des séquences nucléiques correspondant aux gènes appelés ureE, ureF, ureG, ureH, ureI et répondant aux enchaînements nucléotidiques présentés ci-dessous :

```
1                                              31
A CTC TTT AGC ATT TTC TAG GA TTT TTT AGG AGC AAC GCT CTT AGA TCC TTA GTT TTT AGC
--leu phe ser ile phe AMB

61                                              91
TCT CTG ATT TTT TGT TTA TCA AAA AAT TGG GGG CTT TTT TTG TTT TTA TTT TTT GTC AAT

121                                             151
TTA CTA TTT TTC TTT ATG ATT AGC TCA AGC AAC AAA AGT TAT TCG TAA GGT GCG TTT GTT

181                       SD                    211
GTA AAA ATT TTT GTT TGG AAG GAA AAG GCA ATG CTA GGA CTT GTA TTG TTA TAT GTT GGG
         ureI                              Met leu gly leu val leu leu tyr val gly

241                                             271
ATT GTT TTA ATC AGC AAT GGG ATT TGC GGG TTA ACC AAA GTC GAT CCT AAA AGC ACT GCG
ile val leu ile ser asn gly ile cys gly leu thr lys val asp pro lys ser thr ala

301                                             331
GTG ATG AAC TTT TTT GTG GGT GGG CTC TCC ATT ATT TGT AAT GTG GTT GTC ATC ACT TAT
val met asn phe phe val gly gly leu ser ile ile cys asn val val val ile thr tyr

361                                             391
TCC GCT CTC AAC CCT ACA GCC CCT GTA GAA GGT GCT GAA GAT ATT GCT CAA GTA TCA CAC
ser ala leu asn pro thr ala pro val glu gly ala glu asp ile ala gln val ser his

421                                             451
CAT TTG ACT AAT TTC TAT GGG CCA GCG ACT GGG TTA TTG TTT GGT TTC ACC TAC TTG TAT
his leu thr asn phe tyr gly pro ala thr gly leu leu phe gly phe thr tyr leu tyr

481                                             511
GCG GCT ATC AAC CAC ACT TTT GGT TTG GAT TGG AGG CCC TAC TCT TGG TAT AGC TTA TTC
ala ala ile asn his thr phe gly leu asp trp arg pro tyr ser trp tyr ser leu phe

541                                             571
GTA GCG ATC AAC ACG ATT CCT GCT GCG ATT TTA TCC CAC TAT AGC GAT ATG CTT GAT GAC
val ala ile asn thr ile pro ala ala ile leu ser his tyr ser asp met leu asp asp

601                                             631
CAC AAA GTG TTA GGC ATC ACT GAA GGC GAT TGG TGG GCG ATC ATT TGG TTG GCT TGG GGT
his lys val leu gly ile thr glu gly asp trp trp ala ile ile trp leu ala trp gly

661                                             691
GTT TTG TGG CTT ACC GCT TTC ATT GAA AAC ATC TTG AAA ATC CCT TTA GGG AAA TTC ACT
val leu trp leu thr ala phe ile glu asn ile leu lys ile pro leu gly lys phe thr

721                                             751
CCA TGG CTT GCT ATC ATT GAG GGC ATT TTA ACC GCT TGG ATC CCT GCT TGG TTA CTC TTT
pro trp leu ala ile ile glu gly ile leu thr ala trp ile pro ala trp leu leu phe

781                                             811
ATC CAA CAC TGG GTG TGA GAT GAT CAT
ile gln his trp val OPA

782                                             812
TCC AAC ACT GGG TGT GAG ATG ATC ATA GAG CGT TTA ATA GGC AAT CTA AGG GAT TTA AAC
         ureE        Met ile ile glu arg leu ile gly asn leu arg asp leu asn
```

3

```
842                                            871
CCC TTG GAT TTC AGC GTG GAT TAT GTG GAT TTG GAA TGG TTT GAA ACG AGG AAA AAA ATC
pro leu asp phe ser val asp tyr val asp leu glu trp phe glu thr arg lys lys ile

902                                            932
GCT CGC TTT AAA ACC AGG CAA GGC AAA GAC ATA GCC GTA CGC CTT AAA GAC GCT CCC AAG
ala arg phe lys thr arg gln gly lys asp ile ala val arg leu lys asp ala pro lys

962                                            992
TTG GGT TTC TCT CAA GGA GAT ATT TTA TTT AAA GAA GAG AAG GAA ATT ATC GCC GTT AAT
leu gly phe ser gln gly asp ile leu phe lys glu glu lys glu ile ile ala val asn

1022                                           1052
ATC TTG GAT TCT GAA GTC ATT CAC ATC CAA GCT AAG AGC GTG GCA GAA GTA GCG AAA ATA
ile leu asp ser glu val ile his ile gln ala lys ser val ala glu val ala lys ile

1082                                           1112
TGC TAT GAA ATA GGA AAC CGC CAT GCG GCT TTA TAC TAT GGC GAG TCT CAA TTT GAA TTT
cys tyr glu ile gly asn arg his ala ala leu tyr tyr gly glu ser gln phe glu phe

1142                                           1172
AAA ACA CCA TTT GAA AAG CCC ACG CTA GCG TTA CTA GAA AAG CTA GGG GTT CAA AAT CGT
lys thr pro phe glu lys pro thr leu ala leu leu glu lys leu gly val gln asn arg

1202                                           1232
GTT TTA AGT TCA AAA TTG GAT TCC AAA GAA CGC TTA ACC GTG AGC ATG CCC CAT AGT GAG
val leu ser ser lys leu asp ser lys glu arg leu thr val ser met pro his ser glu

1262                                    1292                    S D
CCT AAT TTT AAG GTC TCA CTG GCG AGC GAT TTT AAA GTG GTC ATG AAA TAG AAA AAC AA
pro asn phe lys val ser leu ala ser asp phe lys val val met lys AMB

1321                                           1351
CAA ATG GAT AAA GGA AAA AGC GTG AAA AGC ATT GAA AAA AGC GTG GGT ATG CTC CCA AAA
F   Met asp lys gly lys ser val lys ser ile glu lys ser val gly met leu pro lys

1381                                           1411
ACT CCA AAG ACA GAC AGC AAT GCT CAT GTG GAT AAT GAA TTT CTG ATT CTG CAA GTC AAT
thr pro lys thr asp ser asn ala his val asp asn glu phe leu ile leu gln val asn

1441                                           1471
GAT GCG GTG TTC CCC ATT GGA TCT TAC ACG CAT TCT TTT GGG CTT TTG GCT AGA AAC TTA
asp ala val phe pro ile gly ser tyr thr his ser phe gly leu leu ala arg asn leu

1501                                           1531
CAT CCA GCA AAA AAG GTT ACT AAT AAA GAA AGC GCT TTA AAA TAT TTA AAA GCC AAT CTC
his pro ala lys lys val thr asn lys glu ser ala leu lys tyr leu lys ala asn leu

1561                                           1591
TCT AGC CAG TTC CTT TAC ACG GAA ATG CTG AGC TTG AAA CTC ACC TAT GAA AGC GCT CTC
ser ser gln phe leu tyr thr glu met leu ser leu lys leu thr tyr glu ser ala leu

1621                                           1651
CAA CAA GAT TTA AAA AGG ATC TTA GGG GTT GAA GAA ATC ATT ACG CTA TCC ACA AGC CCC
gln gln asp leu lys arg ile leu gly val glu glu ile ile thr leu ser thr ser pro
```

1681
ATG GAA TTG CGA TTA GCC AAT CAA AAG CTA GGC AAT CGT TTC ATT AAA ACC TTA CAA GCC
met glu leu arg leu ala asn gln lys leu gly asn arg phe ile lys thr leu gln ala

1741                                        1771
ATG AAC GAA TTA GAC ATT GGC GCA TTT TTT AAC GCT TAC GCT CAA CAA ACC GAA GAC CCC
met asn glu leu asp ile gly ala phe phe asn ala tyr ala gln gln thr glu asp pro

1801                                        1831
ACC CAT GCC ACT AGC TAT GGC GTT TTT GCG GCG AGT TTG GGG ATT GAA TTG AAA AAG GCT
thr his ala thr ser tyr gly val phe ala ala ser leu gly ile glu leu lys lys ala

1861                                        1891
TTA AGG CAT TAT CTT TAT GCA CAA ACT TCT AAC ATG GTA ATT AAC TGC GTT AAA AGC GTC
leu arg his tyr leu tyr ala gln thr ser asn met val ile asn cys val lys ser val

1921                                        1951
CCA CTA TCT CAA AAC GAT GGG CAA AAA ATC TTA TTG AGC TTG CAA AGC CCT TTT AAC CAG
pro leu ser gln asn asp gly gln lys ile leu leu ser leu gln ser pro phe asn gln

1981                                        2011
CTC ATA GAA AAA ACC CTA GAA CTA GAC GAA AGC CAC TTG TGC GCG GCA AGC GTT CAA AAC
leu ile glu lys thr leu glu leu asp glu ser his leu cys ala ala ser val gln asn

2041                                        2071
GAC ATT AAG GCG ATG CAG CAT GAG AGT TTA TAC TCG CGC CTT TAT ATG TCT TGA ATT TTA
asp ile lys ala met gln his glu ser leu tyr ser arg leu tyr met ser OPA

2102          SD                            2132
TCT CAA ATT GAA AGG AAT TTT ATG GTA AAA ATT GGA GTT TGT GGT CCT GTA GGA AGC GGT
    ureG                   Met val lys ile gly val cys gly pro val gly ser gly

2162                                        2192
AAA ACC GCC TTG ATT GAA GCT TTA ACG CGC CAC ATG TCA AAA GAT TAT GAC ATG GCG GTC
lys thr ala leu ile glu ala leu thr arg his met ser lys asp tyr asp met ala val

2222                                        2252
ATC ACT AAT GAT ATT TAC ACG AAA GAA GAC GCA GAA TTT ATG TGT AAA AAT TCG GTG ATG
ile thr asn asp ile tyr thr lys glu asp ala glu phe met cys lys asn ser val met

2282                                        2312
CCA CGA GAG AGG ATC ATT GGC GTA GAA ACA GGA GGC TGT CCG CAC ACG GCT ATT AGA GAA
pro arg glu arg ile ile gly val glu thr gly gly cys pro his thr ala ile arg glu

2342                                        2372
GAC GCT TCT ATG AAT TTA GAA GCC GTA GAA GAA ATG CAT GGC CGT TTC CCT AAT TTG GAA
asp ala ser met asn leu glu ala val glu glu met his gly arg phe pro asn leu glu

2402                                        2432
TTG CTT TTG ATT GAA AGC GGA GGC AGT AAC CTT TCA GCG ACT TTC AAC CCA GAG CTA GCG
leu leu leu ile glu ser gly gly ser asn leu ser ala thr phe asn pro glu leu ala

2462                                        2492
GAC TTT ACG ATC TTT GTG ATT GAT GTG GCT GAG GGC GAT AAA ATC CCC AGA AAA GGC GGG
asp phe thr ile phe val ile asp val ala glu gly asp lys ile pro arg lys gly gly

5

```
2522                                            2552
CCA GGA ATC ACG CGT TCA GAC TTG CTT GTC ATC AAT AAG ATT GAT TTA GCC CCC TAT GTG
pro gly ile thr arg ser asp leu leu val ile asn lys ile asp leu ala pro tyr val

2582                     .                      2612  .
GGA GCC GAC TTG AAA GTC ATG GAA AGG GAT TCT AAA AAA ATC GCG GCG AAA AGC CCT TTA
gly ala asp leu lys val met glu arg asp ser lys lys ile ala ala lys ser pro leu

2642      .                                     2672      -
TTT TTA CCG AAT ATC CGC GCT AAA GAA GGT TTA GAC GAT GTG ATC GCT TGG ATC AAG CGC
phe leu pro asn ile arg ala lys glu gly leu asp asp val ile ala trp ile lys arg

2702
AAC GCT TTA TTG GAA GAT TGA TGA ACA CTT
asn ala leu leu glu asp OPA

2701                 S D                        2731 .
CAA CGC TTT ATT GGA AGA TTG ATG AAC ACT TAC GCT CAA GAA TCC AAG CTC AGG TTA AAA
        ureH                    Met asn thr tyr ala gln glu ser lys leu arg leu lys

2761                                            2791
ACC AAA ATA GGG GCT GAC GGG CGG TGC GTG ATT GAA GAC AAT TTT TTC ACG CCC CCC TTT
thr lys ile gly ala asp gly arg cys val ile glu asp asn phe phe thr pro pro phe

2821                                            2851
AAG CTC ATG GCG CCC TTT TAC CCT AAA GAC GAT TTA GCG GAA ATC ATG CTT TTA GCG GTA
lys leu met ala pro phe tyr pro lys asp asp leu ala glu ile met leu leu ala val

2881                                            2911
AGC CCT GGC TTA ATG AAA GGC GAT GCA CAA GAT GTG CAA TTG AAC ATC GGT CCA AAT TGC
ser pro gly leu met lys gly asp ala gln asp val gln leu asn ile gly pro asn cys

2941                                            2971
AAG TTA AGG ATC ACT TCG CAA TCC TTT GAA AAA ATC CAT AAC ACT GAA GAC GGG TTT GCT
lys leu arg ile thr ser gln ser phe glu lys ile his asn thr glu asp gly phe ala

3001                                            3031  .
AGC AGA GAC ATG CAT ATC GTT GTG GGG GAA AAC GCT TTT TTA GAC TTC GCG CCC TTC CCG
ser arg asp met his ile val val gly glu asn ala phe leu asp phe ala pro phe pro

3061                                            3091
TTA ATC CCC TTT GAA AAC GCG CAT TTT AAG GGC AAT ACC ACG ATT TCT TTG CGC TCT AGC
leu ile pro phe glu asn ala his phe lys gly asn thr thr ile ser leu arg ser ser

3121                                            3151
TCC CAA TTG CTC TAT AGT GAA ATC ATT GTC GCA GGG CGA GTG GCG CGC AAT GAG TTG TTT
ser gln leu leu tyr ser glu ile ile val ala gly arg val ala arg asn glu leu phe

3181          .                                3211
AAA TTC AAC CGC TTG CAC ACC AAA ATC TCT ATT TTA CAA GAT GAG AAA CCC ATC TAT TAT
lys phe asn arg leu his thr lys ile ser ile leu gln asp glu lys pro ile tyr tyr

3241                                            3271
GAC AAC ACG ATT TTA GAT CCC AAA ACC ACC GAC TTA AAT AAC ATG TGC ATG TTT GAT GGC
asp asn thr ile leu asp pro lys thr thr asp leu asn asn met cys met phe asp gly
```

```
3301                                          3331
TAT ACG CAT TAT TTG AAT TTG GTG CTG GTC AAT TGC CCC ATA GAG CTG TCT GGC GTG CGA
tyr thr his tyr leu asn leu val leu val asn cys pro ile glu leu ser gly val arg

3361                                          3391
GGA TTG ATT GAA GAG AGC GAA GGA GTG GAT GGA GCC GTG AGT GAA ATC GCT AGT TCT CAT
gly leu ile glu glu ser glu gly val asp gly ala val ser glu ile ala ser ser his

3421                                          3451
TTA TGC CTG AAA GCT TTA GCG AAA GGC TCA GAA CCC TTG TTG CAT TTA AGA GAA AAA ATC
leu cys leu lys ala leu ala lys gly ser glu pro leu leu his leu arg glu lys ile

3481                                          3511
GCT CGC TTT ATC ACG CAA ACG ATT ACG CCA AAG GTT TAA AAA ACA CTT TAA AAA AGA TTA
ala arg phe ile thr gln thr ile thr pro lys val OCH

3541
TAC CCT TTA GTC TTT TTT AA
```

ou, toute partie d'au moins une de ces séquences nucléiques.

Une séquence nucléotidique selon l'invention est constituée soit par de l'ADN, soit par de l'ARN.

L'invention concerne aussi une séquence nucléotidique modifiée par rapport à la séquence nucléotidique décrite ci-dessus, par délétion, addition, substitution ou inversion d'un ou plusieurs nucléotides, de telle façon que les propriétes fonctionnelles des polypeptides codés par ces gènes modifiés sont soit conservées soit atténuées, voire supprimées, par rapport aux propriétés des polypeptides UreE, UreF, UreG, UreH ou UreI tels qu'exprimés par H. pylori, ou de telle façon que cette séquence n'exprime pas de polypeptide chez H. pylori.

Selon un mode particulier de réalisation de l'invention et dans le cadre de la définition précédente, une séquence nucléotidique est caractérisée en ce qu'elle est constituée par, ou en ce qu'elle comprend :

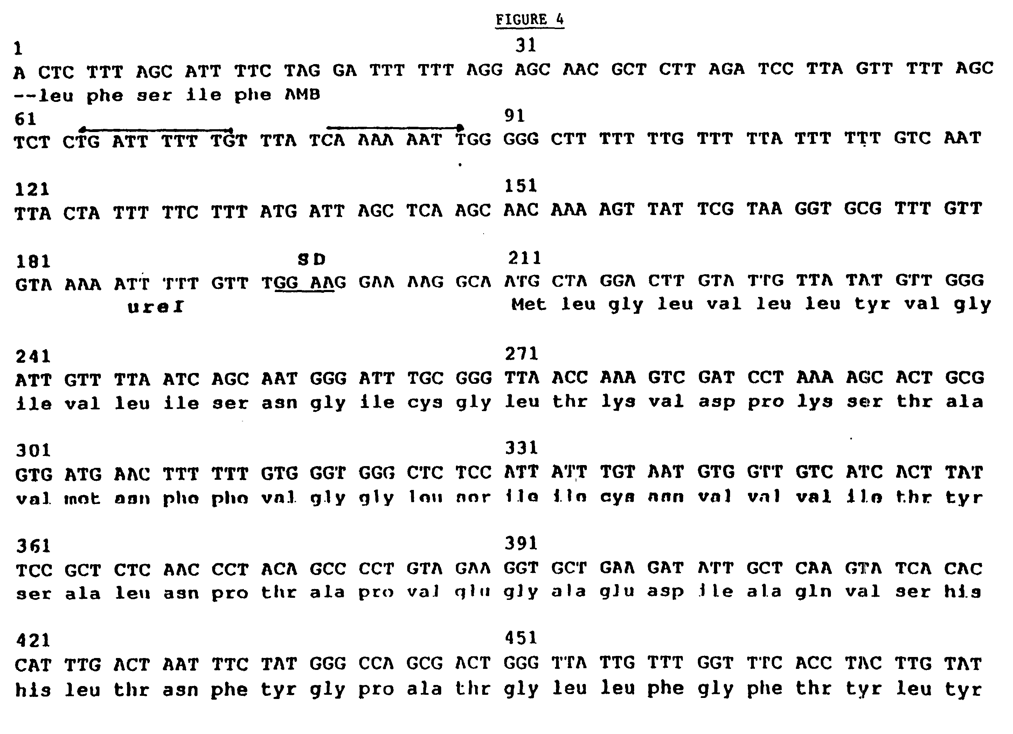

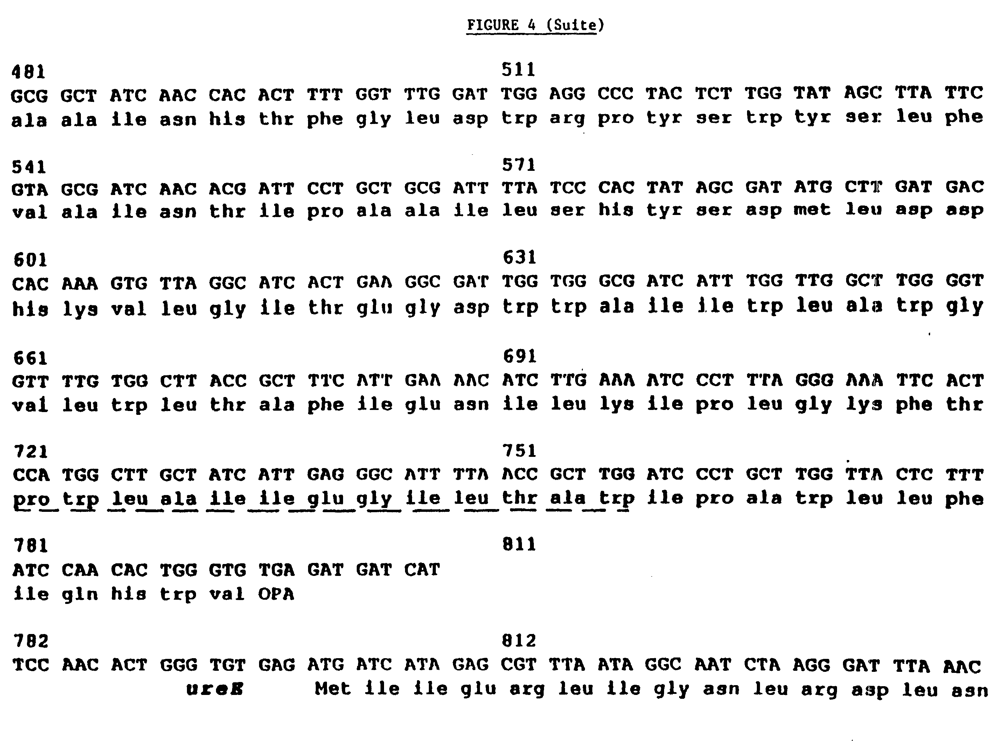

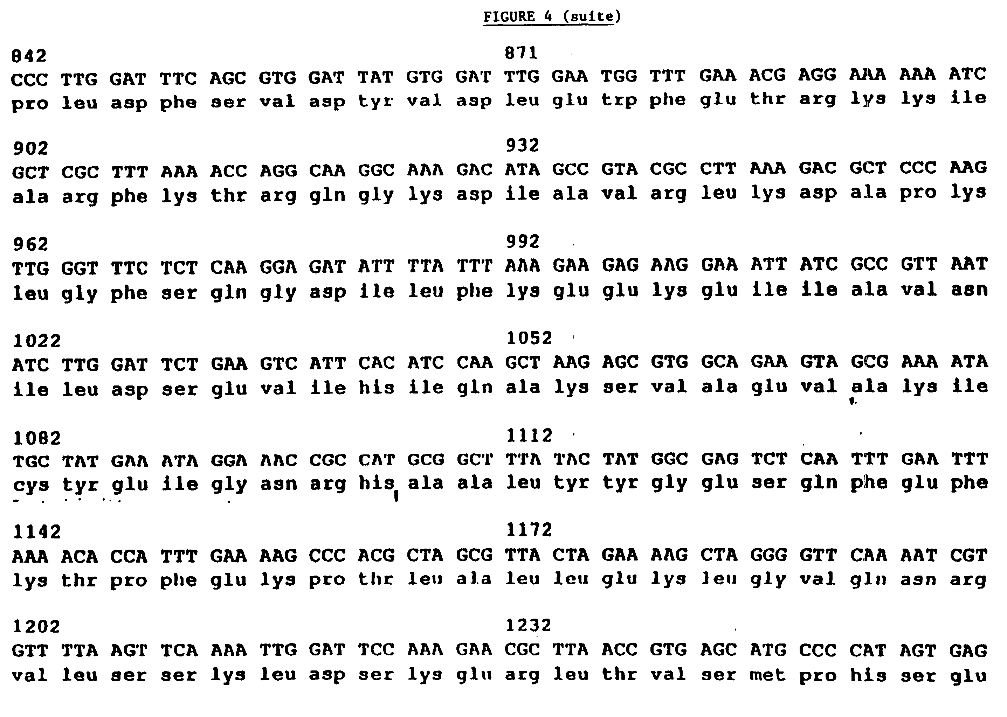

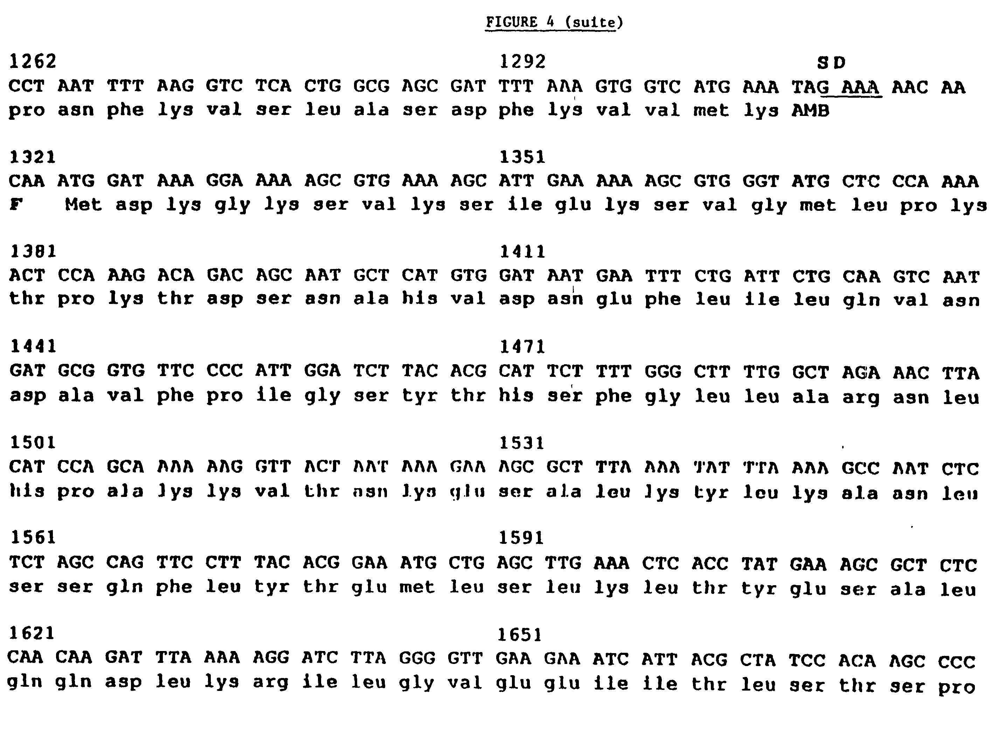

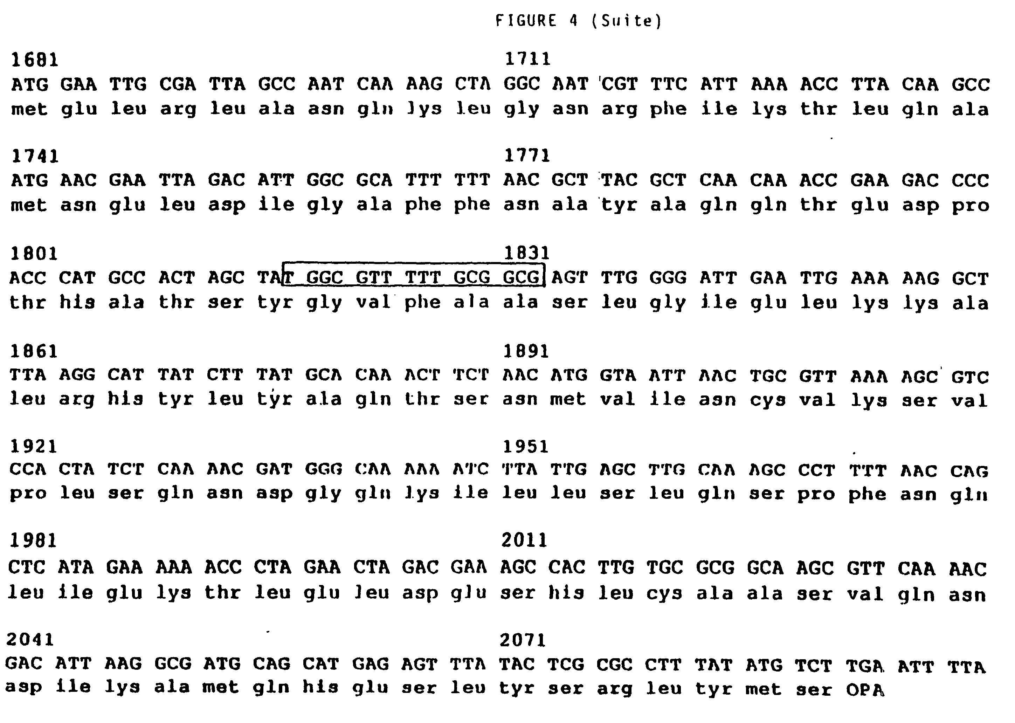

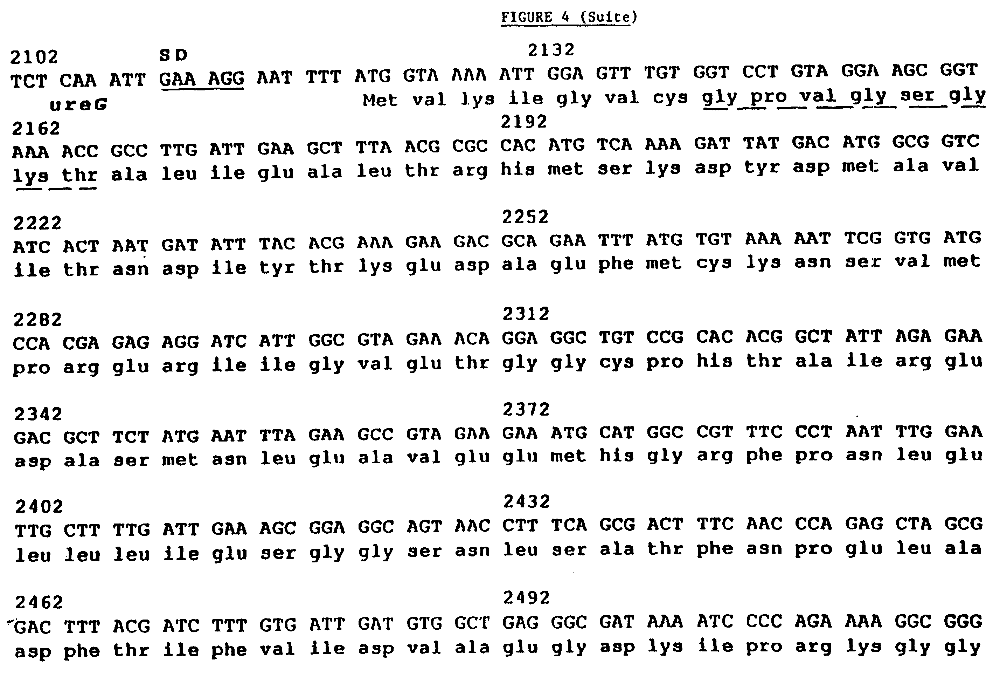

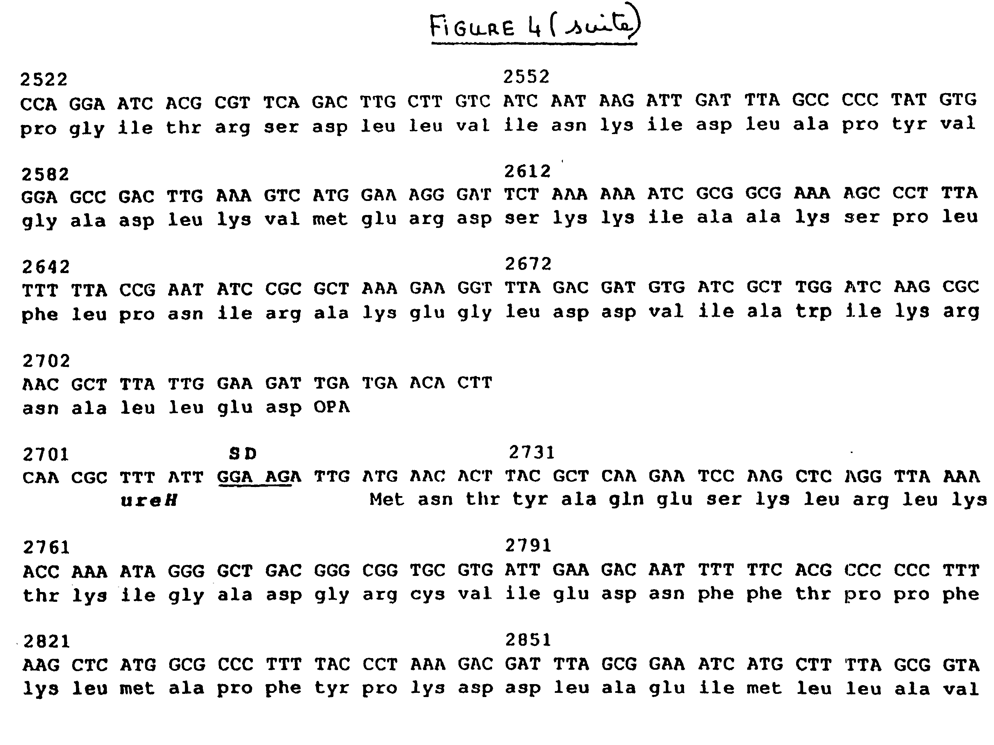

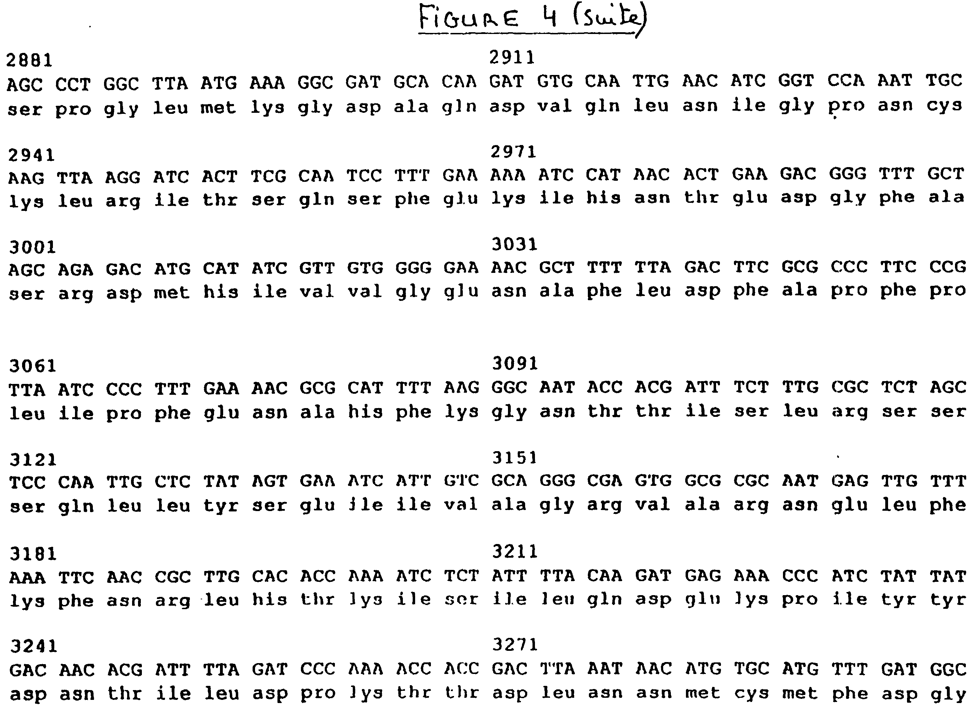

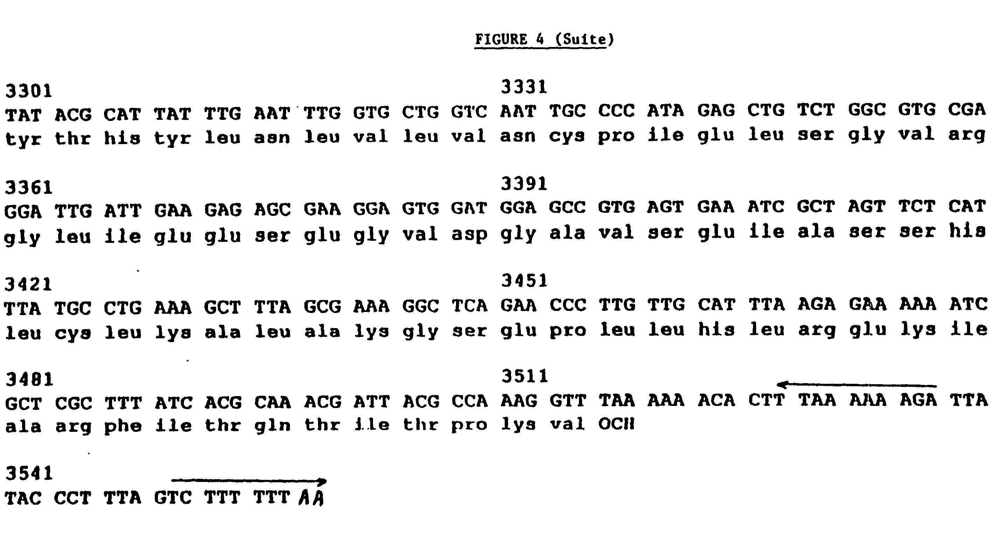

a) l'ensemble des séquences nucléiques correspondant aux gènes appelés ureE, ureF, ureG, ureH, ureI et répondant aux enchaînements nucléotidiques présentés à la figure 4 ou,

b) l'ensemble formé par les séquences nucléiques (variantes) correspondant à ces gènes modifiés indépendamment les uns des autres , de telle façon que l'ensemble de ces variantes code pour des polypeptides ayant une homologie fonctionnelle avec les polypeptides UreE, UreF, UreG, UreH ou UreI tels qu'exprimés par H.pylori, ou au contraire code pour des polypeptides modifiés, pour atténuer voire supprimer les propriétés fonctionnelles des polypeptides UreE, UreF, UreG, UreH ou UreI tels qu'exprimés par H. pylori.

Des fragments (enchaînements nucléotidiques) des séquences nucléotidiques ci-dessus sont intéressants pour différentes raisons et à titre d'exemple on peut définir :

- des fragments des susdites séquences, ayant conservé la capacité de coder pour des polypeptides ayant une homologie fonctionnelle avec les polypeptides tels qu'obtenus par expression d'un gène choisi parmi ureE, ureF, ureG ou ureI, dans H. pylori ;
- des fragments codant pour toute partie des polypeptides ci-dessus tels qu'obtenus chez H. pylori, et en particulier codant pour des peptides ou des parties de polypeptides reconnus par des anticorps dirigés contre H. pylori ou capables de se comporter comme des haptènes ou des immunogènes ;
- des fragments des susdites séquences dépourvus de la capacité de coder pour les polypeptides de H. pylori tels qu'exprimés à partir des gènes ureE, ureF, ureG, ureH ou ureI ;
- des fragments codant pour des polypeptides ou peptides ayant des propriétés atténuées, voire supprimées par rapport aux propriétés des polypeptides codés par les gènes ureE, ureF, ureG, ureH ou ureI de H. pylori.

De tels fragments ont avantageusement au moins 15 nucléotides, de préférence au moins 20 nucléotides.

Ces gènes ureE, ureF, ureG, ureH et ureI, sont présents sur un chromosome de H. pylori; ces gènes sont des gènes dits accessoires par rapport aux gènes de structure de l'uréase (ureA, ureB). Par opposition aux gènes de structure, les gènes accessoires ne sont pas nécessaires à la formation de l'enzyme uréase. En revanche ils interviennent dans l'expression fonctionnelle de l'uréase telle qu'exprimée chez H. pylori, par des moyens de régulation et/ou de maturation de l'uréase formée. L'uréase est en effet exprimée sous la forme d'une apoenzyme inactive avant de subir une étape de maturation au sein de H. pylori, étape qui lui confère sa forme d'enzyme fonctionnelle.

Les inventeurs ont par ailleurs constaté que la présence de ces cinq gènes accessoires est indispensable à l'expression de l'uréase fonctionnelle dans des cellules de E.coli préalablement transformées avec les gènes de structure ureA, ureB, ureC et ureD.

En conséquence l'identification de ces gènes et de leurs séquences nucléotidiques, permet d'envisager des moyens pour moduler l'activité uréasique dans des souches de H. pylori en particulier pour préparer des souches atténuées.

Selon un premier mode de réalisation de l'invention, des séquences nucléotidiques intéressantes codent pour des polypeptides ayant une homologie fonctionnelle avec les polypeptides UreE, UreF, UreG, UreH et UreI naturels. Cette homologie entre des polypeptides, est appréciée par rapport à la capacité de ces polypeptides, de fonctionner au sein de H. pylori, comme les polypeptides UreE, UreF, UreG, UreH et UreI naturels et par conséquent de contribuer à la formation de l'uréase fonctionnelle à partir de l'apoenzyme.

Cette homologie fonctionnelle peut être détectée par la mise en oeuvre du test suivant : $10^9$ bactéries sont resuspendues dans 1 ml de milieu urée-indole et incubées à 37°C. L'hydrolyse de l'urée conduit à la libération d'ammoniaque, qui en augmentant le pH, induit un changement de coloration de orange à rouge fushia.

Au contraire on peut mettre en oeuvre dans le cadre de l'invention, des séquences nucléotidiques, répondant à l'ensemble des séquences nucléiques correspondant aux gènes ureE, ureF, ureG, ureH ou ureI, ces séquences étant modifiées de façon à ce que les polypeptides pour lesquels elles codent ne possèdent plus la capacité des polypeptides naturels de permettre la production d'une uréase fonctionnelle dans H. pylori ou le cas échéant dans une autre espèce. Dans ce cas on cherche à atténuer ou à supprimer les propriétés fonctionnelles des polypeptides naturels, tels qu'exprimés par H. pylori. on considère que les propriétés fonctionnelles sont atténuées lorsque la souche dans laquelle les séquences nucléotidiques selon l'invention sont insérées, produit une uréase non pathogène par exemple sous la forme d'une apoenzyme. Cette pathogénicité peut être évaluée par la mise en oeuvre du test suivant :

On teste l'implantation dans l'estomac d'un animal, de préférence le porcelet gnotobiotique, de la souche recombinante, en utilisant la technique décrite par Eaton et al (1991 Infect. Immun. 59: 2470-2475).

Selon un premier mode de réalisation de l'invention, une séquence nucléotidique telle que définie précédemment peut être associée aux séquences nucléiques correspondant aux gènes de structure ureA et ureB codant pour les sous-unités uréasiques chez H. pylori.

Selon un autre mode de réalisation de l'invention, cette séquence nucléotidique est associée aux gènes ureA, ureB, ureC et/ou ureD codant pour l'uréase chez H. pylori.

Dans ce cas les différents gènes peuvent être localisés sur des réplicons distincts.

L'invention concerne également les séquences nucléotidiques entrant dans le cadre de la définition précédente et répondant à l'un des enchaînements nucléotidiques codant correspondant aux gènes ureE, ureF, ureG, ureH ou ureI. A cet égard l'invention vise en particulier les enchaînements suivants :

- l'enchaînement ureE correspondant aux nucléotides 800 à 1309 de la séquence de la figure 4, ou tout fragment de cet enchaînement dès lors qu'il hybride dans des conditions stringentes c'est à dire à 68°C dans 6 x SSC milieu de Denhard ou à 37°C dans 5 x SSC 50% Formamide avec l'enchaînement ureE ou avec la séquence complémentaire à cet enchaînement,

- l'enchaînement ureF correspondant aux nucléotides 1324 à 2091 de la séquence de la figure 4, ou tout fragment de cet enchaînement dès lors qu'il hybride dans des conditions stringentes c'est à dire à 68°C dans 6 x SSC milieu de Denhard ou à 37°C dans 5 x SSC 50% Formamide avec l'enchaînement ureF ou avec la séquence complémentaire à cet enchaînement,

- l'enchaînement ureG correspondant aux nucléotides 2123 à 2719 de la séquence de la figure 4, ou tout fragment de cet enchaînement dès lors qu'il hybride dans des conditions stringentes c'est à dire à 68°C dans 6 x SSC milieu de Denhard ou à 37°C dans 5 x SSC 50% Formamide avec l'enchaînement ureG ou avec la séquence complémentaire à cet enchaînement,

- l'enchaînement ureH correspondant aux nucléotides 2722 à 3516 de la séquence de la figure 4, ou tout fragment de cet enchaînement dès lors qu'il hybride dans des conditions stringentes c'est à dire à 68°C dans 6 x SSC milieu de Denhard ou à 37°C dans 5 x SSC 50% Formamide avec l'enchaînement ureH ou avec la séquence complémentaire à cet enchaînement,

- l'enchaînement ureI correspondant aux nucléotides 211 à 795 de la séquence de la figure 4, ou tout fragment de cet enchaînement dès lors qu'il hybride dans des conditions stringentes c'est à dire à 68°C dans 6 x SSC milieu de Denhard ou à 37°C dans 5 x SSC 50% Formamide avec l'enchaînement ureI ou avec la séquence complémentaire à cet enchaînement.

On appelle ici "séquences complémentaires" en ce qui concerne les séquences d'ADN, des séquences inverses et complémentaires. Le terme "inverse" rend compte de la restauration de l'orientation 5'-3' de l'acide nucléique, complémentaire par la nature des nucléotides et ce par rapport à une séquence donnée.

L'invention vise aussi un enchaînement nucléotidique particulier répondant à la séquence suivante :

**GCG AAA ATA TGC TAT GAA ATA GGA AAC CGC CAT**

L'invention se rapporte également à toute séquence d'ADN qui comprend cet enchaînement nucléotidique.

Les séquences nucléotidiques selon l'invention répondant aux définitions précédentes peuvent entrer dans la constitution de sondes, lorsqu'elles sont marquées, par exemple à leur extrémité 5' et/ou 3', par une substance que l'on peut détecter. A titre de marqueur on peut citer les isotopes radioactifs, les enzymes, les marqueurs chimiques ou chimioluminescents, les fluorochromes, les haptènes ou les anticorps, des analogues de base ou encore des marqueurs physiques. Ces marqueurs peuvent le cas échéant être fixés à un support solide par exemple un support particulaire ou membranaire, comme des billes magnétiques.

A titre de marqueur préféré, on peut citer le phosphore radioactif ($^{32}$p) incorporé à l'extrémité 5' de la séquence utilisée comme sonde.

Avantageusement une sonde nucléotidique selon l'invention comprend tout fragment des gènes décrits, par exemple des fragments d'environ 45 nucléotides.

Des sondes préférées selon l'invention sont constituées par des fragments issus du gène ureH ou de préférence du gène ureI.

A partir des séquences nucléotidiques selon l'invention, on peut aussi définir des amorces (primers) utilisables pour la détection in vitro d'une infection par H. pylori. Une amorce est caractérisée en ce qu'elle comprend un fragment nucléotidique tel qu'issu d'une séquence décrite précédemment, comprenant d'environ 18 à environ 30 de préférence d'environ 25 à environ 30 nucléotides. Une telle amorce peut être mise en oeuvre dans des réactions d'amplification génique, par exemple selon une technique de polymérisation en chaîne.

Pour l'utilisation dans une technique d'amplification, des amorces de l'invention sont prises en combinaison deux à deux, de façon à hybrider dans des conditions déterminées avec les extrémités 5' et 3' respectives du fragment nucléotidique à amplifier.

Si l'on met en oeuvre la technique PCR, les conditions requises pour l'hybridation spécifique des amorces avec l'ADN à détecter sont les conditions décrites dans les demandes EP 200363, 201184, 229701 et la température est calculée selon la formule

$$T\ (^{\circ}C) = [4(C + G) + 2\ (A + T) - 10]$$

dans laquelle A, T, C, G représentent respectivement le nombre de nucléotides A, T, C, G dans les amorces utilisées.

Les techniques d'amplification utilisables dans le cadre de l'invention comportent par exemple la technique PCR (Polymerase Chain Reaction) décrite dans les demandes de brevet européen de Cetus (n° 200363, 201184 et 229701), ou encore la technique de "Qβ Replicase" décrite dans Biotechnology (Vol. 6, Octobre 1988).

D'autres séquences nucléotidiques selon l'invention sont des séquences hybridant dans des conditions stringentes telles que définies ci-dessus avec une séquence définie dans les pages précédentes ou une séquence complémentaire de ces séquences.

Les séquences nucléotidiques et les vecteurs de l'invention peuvent aussi être utilisés pour l'expression d'autres gènes ou séquences de H. pylori ou d'autres souches dans H. pylori ou dans d'autres hôtes comme E.coli, l'Adenovirus.

L'invention vise en outre un polypeptide caractérisé en ce qu'il correspond à l'un des polypeptides UreE, UreF, UreG, UreH ou UreI présentés à la figure 4, à toute partie d'au moins un de ces polypeptides. L'invention vise en particulier tout polypeptide modifié dès lors qu'il présente une homologie fonctionnelle avec le polypeptide d'origine UreE, UreF, UreG, UreH ou UreI tel qu'exprimé par H. pylori, ou au contraire modifié par délétion, addition, substitution ou inversion d'un ou plusieurs acides aminés, pour atténuer voire supprimer ses propriétés fonctionnelles s'agissant de l'activité uréasique telle qu'exprimée par H. pylori.

Les polypeptides UreE, UreF, UreG, UreH et UreI interviennent notamment dans la régulation et la maturation de l'uréase chez H. pylori.

Un autre polypeptide selon l'invention est celui qui répond à l'enchaînement de 11 acides aminés suivant :

**Ala Lys Ile Cys Tyr Glu Ile Gly Asn Arg His**

Les polypeptides de l'invention et en particulier le polypeptide dont la séquence est donnée ci-dessus, peuvent être utilisés pour la production d'anticorps polyclonaux ou monoclonaux, ou pour la détection d'anticorps dans un échantillon biologique infecté par H. pylori.

Des anticorps monoclonaux peuvent être préparés par la technique des hybridomes ou par les techniques connues pour préparer des anticorps humains.

Ces anticorps peuvent également être préparés selon la technique décrite par Marks et al (J. Mol. Biol. 1991 222,581-597).

L'invention vise aussi des anticorps anti-idiotypiques.

Des anticorps contre l'enchaînement de 11 acides aminés ci-dessus pourraient être mis en oeuvre dans le cadre d'une réaction de blocage de la maturation de l'uréase.

L'invention concerne en outre l'utilisation des anticorps monoclonaux ou polyclonaux, dans des compositions pour traiter une infection par H. pylori.

L'invention a également pour objet des vecteurs recombinants caractérisés en ce qu'ils contiennent une séquence d'ADN de l'invention. De tels vecteurs recombinants peuvent par exemple être des cosmides ou des plasmides.

Un vecteur particulièrement avantageux pour la réalisation de l'invention est caractérisé en ce qu'il s'agit du plasmide pILL753 contenu dans E.coli HB101 déposé à la CNCM (Collection Nationale de Cultures de Microorganismes, Paris France) le 3 Octobre 1991 sous le numéro I-1148.

Un autre vecteur recombinant particulièrement avantageux est caractérisé en ce qu'il s'agit du plasmide pILL763 contenu dans E.coli HB101 déposé à la CNCM le 3 Octobre 1991 sous le numéro I-1149.

L'invention a aussi pour objet un hôte cellulaire recombinant (ou souche cellulaire recombinante), caractérisé en ce qu'il est transformé par une séquence nucléotidique répondant aux définitions précédemment données. Cet hôte cellulaire ainsi transformé doit permettre l'expression de la séquence nucléotidique des gènes accessoires de l'uréase, le cas échéant modifiés conformément aux définitions précédentes.

A titre préféré, un hôte cellulaire recombinant est une souche de H. pylori modifiée par une des séquences nucléotidiques précédemment définies, et de façon avantangeuse modifiée de telle façon que les produits des gènes accessoires modifiés qu'elle exprime, contribuent à atténuer les effets de l'uréase, en particulier ses effets pathogènes.

Par exemple une telle souche recombinante peut être obtenue par mutation de la souche N6 de H. pylori déposée à la NCIMB (National Collections of Industrial and Marine Bacteria LTD) en Grande Bretagne, le 26 Juin 1992, sous le numéro NCIMB 40512, la mutation étant effectuée au niveau de l'un au moins des gènes ureE, ureF, ureG, ureH ou ureI, et/ou au niveau d'un ou plusieurs des gènes de structure, par exemple ureA ou ureB.

De préférence on formera dans le cadre de l'invention, des souches recombinantes et en particulier des souches de H. pylori recombinantes dont l'activité uréasique est atténuée conformément aux critères déterminés précédemment.

Ainsi des souches N6 recombinantes particulièrement avantageuses sont celles qui permettent d'obtenir un phénotype uréase-négatif et comportent au moins un des gènes ureE, ureF, ureG, ureH ou ureI muté.

Une inactivation du gène ureI permet par exemple de préparer des souches H. pylori uréase-négatives. De même certaines mutations au sein de ureI permettent d'obtenir un phénotype uréase-négatif chez H. pylori, alors que les produits des gènes ureA et ureB sont exprimés. Il s'agit par exemple de la mutation n°8 décrite dans les exemples.

Une autre mutation particulièrement intéressante, notamment pour la préparation de souches vaccinantes, et en particulier de souches H. pylori vaccinantes, est une mutation du gène ureG. Une souche H. pylori recombinante, dans laquelle le gène ureG est muté, présente les propriétés suivantes :

- la souche ainsi mutée conserve la capacité de déclencher une réponse immunitaire ;
- la souche ainsi mutée est dépourvue d'activité uréasique.

On peut cependant transformer d'autres souches avec les séquences de l'invention. En particulier on aura recours à E.coli pour réaliser des mutations dans les gènes ureE, ureF, ureG, ureH ou ureI, préalablement insérés dans cette souche, par exemple par l'intermédiaire d'un plasmide. Les gènes ainsi mutés peuvent ensuite être introduits dans une autre cellule hôte, par exemple dans H. pylori pour permettre un remplacement allélique et créer une mutation.

On note que la délétion du gène ureI dans une cellule E.coli recombinante selon l' invention n'altère pas le phénotype uréase-positif dès lors que les autres conditions pour l'expression de ce phénotype sont réunies.

La souche E.coli recombinante peut par ailleurs être utilisée pour produire les polypeptides UreE, UreF, UreG, UreH ou UreI et les purifier par les techniques classiques.

Les souches recombinantes de H. pylori, à activité uréase atténuée, peuvent être également utilisées pour le transport et l'expression de gènes hétérologues, par exemple des gènes du choléra ou des salmonelles

Différentes techniques peuvent être employées pour réaliser des souches recombinantes. On aura par exemple recours à la technique d'électroporation telle que décrite dans les exemples de cette demande.

Le cas échéant cette technique d'électroporation peut être modifiée en supprimant l'étape consistant à effectuer un choc électrique au niveau des cellules à transformer.

L'invention propose des moyens pour protéger contre une infection par H. pylori et en particulier par l'administration de compositions immunogènes contenant une souche cellulaire recombinante caractérisée par une activité uréasique atténuée. De telles compositions immunogènes peuvent être utilisées en médecine humaine.

Une composition immunogène peut contenir des souches telles que des cellules de H. pylori dont l'activité uréasique est atténuée par insertion dans la souche d'une séquence nucléotidique selon l'invention, comportant au moins une séquence correspondant aux gènes ureE, ureF, ureG, ureH ou ureI, le cas échéant modifiés pour diminuer l'activité uréasique.

Il peut s'agir de façon générale de tout hôte capable de produire une uréase atténuée, par exemple par mutation des séquences nucléotidiques d'un ou plusieurs gènes ureA, ureB, ureC, ureD, ureE, ureF, ureG, ureH ou ureI ou par expression d'une forme tronquée d'un polypeptide intervenant dans la structure, la maturation ou la régulation de l'uréase.

L'invention a aussi pour objet un kit pour le diagnostic in vitro d'une infection par H. pylori sur un échantillon biologique déterminé, caractérisé en ce qu'il comprend :

- au moins un couple d'amorces nucléotidiques répondant aux critères ci-dessus, capables d'hybrider aux extrémités 5' et en 3' d'un fragment nucléotidique spécifique d'au moins une séquence nucléique correspondant à un gène choisi parmi ureE, ureF, ureG, ureH ou ureI,
- des réactifs nécessaires à l'extraction des acides nucléiques à partir de l'échantillon traité,
- des réactifs pour effectuer la polymérisation dudit fragment nucléotidique, à partir des amorces nucléotidiques, notamment des enzymes de polymérisation, en quantité suffisante pour réaliser l'amplification du fragment que l'on souhaite amplifier,
- au moins un enchaînement de nucléotides pouvant être utilisé comme sonde et capable d'hybrider dans des conditions déterminées avec le fragment d'ADN amplifié,
- le cas échéant des moyens pour révéler l'hybridation.

Selon un mode de réalisation particulier de l'invention, on peut également incorporer au kit,

- un contrôle interne de la réaction d'amplification par exemple constitué par un acide nucléique éventuellement porté par un plasmide, ledit acide nucléique pouvant aisément être détecté par hybridation, par exemple du fait qu'il contient un gène de résistance à un antibiotique, ou du fait qu'il est constitué par de l'ADN chromosomique de N6, ledit fragment étant en outre muni à ces deux extrémités d'au moins une amorce d'amplification, ces amorces étant ou non choisies parmi les amorces de l'invention, et
- une sonde capable d'hybrider avec l'acide nucléique contenu dans le contrôle interne,
- le cas échéant, une réverse transcriptase pour obtenir de l'ADNc à partir de l'ARN éventuellement présent dans l'échantillon testé.

La présence d'un contrôle interne ajouté à l'échantillon permet de détecter la présence de "faux négatifs" parmi les échantillons. En effet, lorsque la sonde spécifique du contrôle interne ne détecte pas un produit d'amplification, on est vraisemblablement en présence d'un échantillon contenant un inhibiteur de la Taq polymérase, inhibiteur qui gêne l'amplification d'ADN ou d'ADNc de H. pylori. Dans ce cas, différentes dilutions de l'échantillon testé peuvent permettre de mettre en évidence la présence d'acide nucléique de H. pylori.

Lorsque le témoin interne présente une réaction positive, une réaction négative au niveau de l'échantillon testé permet de déduire qu'il y a bien absence de H. pylori.

On note que les amorces incorporées au contrôle interne, ne sont pas nécessairement celles de l'invention. Cependant, le choix d'autres amorces peut entraîner une diminution de sensibilité.

A titre d'exemple d'échantillon biologique pour la détection d'une infection chez l'homme par H. pylori, on utilisera des prélèvements tels que des biopsies, du jus gastrique ou éventuellement de la salive ou des selles.

Ce kit peut aussi être mis en oeuvre pour des contrôles de pollution des eaux ou des contrôles sur des aliments.

L'invention se rapporte aussi à un procédé pour le diagnostic in vitro d'une infection par H. pylori, dans un échantillon biologique déterminé, caractérisé en ce qu'il comprend les étapes de :

a) mise en contact de l'acide nucléique de l'échantillon susceptible de contenir H. pylori dans des conditions permettant l'accessibilité sous forme d'ADN simple brin ou d'ARN, avec au moins un couple d'amorces nucléotidiques selon l'invention, lesdites amorces pouvant hybrider avec l'acide nucléique de H. pylori s'il est présent, et initier la synthèse du produit d'élongation desdites amorces, chaque brin de séquence nucléotidique de H. pylori servant de matrice lorsqu'il est apparié avec les amorces;

b) séparation des brins d'acide nucléique synthétisés, de leur matrice;

c) répétition de la synthèse du produit d'élongation, à partir de chaque brin d'acide nucléique présent à l'issue de l'étape b) et susceptible d'hybrider avec les amorces, jusqu'à l'obtention d'une amplification de l'acide nucléique recherché, suffisante pour être détectée,

d) mise en contact du produit de l'étape c) avec une sonde nucléotidique dans des conditions permettant de détecter la présence de l'acide nucléique amplifié recherché;

e) détection des produits de l'hybridation éventuellement formés.

Selon un mode de réalisation préféré du procédé pour le diagnostic in vitro ci-dessus défini, la mise en contact de l'échantillon testé est précédée d'une étape de traitement de l'échantillon de façon à en extraire l'acide nucléique.

Selon un autre mode de réalisation préféré, le procédé comporte une étape préalable à la mise en contact avec les amorces consistant en un traitement de l'acide nucléique de l'échantillon avec une réverse transcriptase, pour obtenir la synthèse d'ADNc à partir de l'ARN éventuellement présent dans l'échantillon testé.

L'invention vise aussi un kit pour le diagnostic in vitro d'une infection par H. pylori caractérisé en ce qu'il comprend :

- une quantité déterminée de sondes selon la définition précédente,
- un milieu approprié pour la réalisation d'une réaction d'hybridation entre l'acide nucléique de H. pylori à détecter et la sonde,
- des réactifs pour la détection des hybrides éventuellement formés.

Un procédé pour l'utilisation de ce kit et pour le diagnostic in vitro d'une infection par H. pylori, à partir d'un échantillon biologique, est caractérisé en ce qu'il comprend :

- la mise en contact de l'échantillon à tester dont l'ADN et/ou de l'ARN a été préalablement rendu accessible, avec une sonde précédemment définie, dans des conditions permettant l'hybridation de l'acide nucléique avec la sonde;
- la mise en évidence d'une réaction d'hybridation éventuelle entre l'acide nucléique et la sonde.

Les séquences nucléotidiques de l'invention peuvent être obtenues soit par extraction de l'acide nucléique de H. pylori et digestion avec des endonucléases choisies et purification, ou encore par synthèse chimique.

A titre d'exemple, on peut citer pour la synthèse de tels fragments d'acides nucléiques, la méthode au phosphotriester, telle que décrite par Narang, S.A. et al dans Meth. of Enzymol., 68, 90 (1979). Une autre méthode adaptée pour la préparation de fragments de nucléotides est la méthode au phosphotriester telle que décrite par Brown E.L. et al, dans dans Meth. of Enzymol., 68, 109 (1979).

Cette préparation peut également être effectuée par un processus automatisé, par exemple faisant intervenir les diethylphosphoramidites en tant que constituants de départ, et dans ce cas, la synthèse peut être réalisée suivant la description de Beaucage et al, Tetrahedron Letters (1981), 22, 1859-1862.

D'autres avantages et propriétés de l'invention apparaissent dans les exemples qui suivent et dans les figures.

## FIGURES

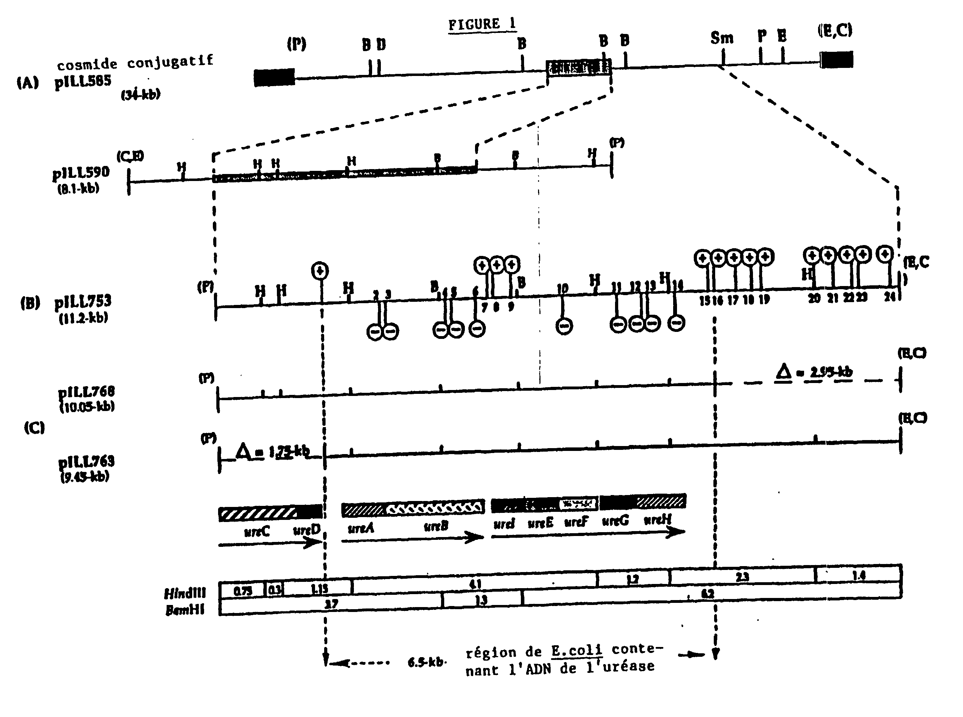

Figure 1: **Sous-clonage et mutagénèse par transposon de pILL753**

A : Carte de restriction linéaire du cosmide hybride pILL585 et du plasmide pILL590 (Labigne et al - 1991). Les cadres gris représent le fragment d'ADN requis pour l'expression de l'uréase dans C. jejuni.

B : Insertion au hasard du transposon MiniTn3-Km. Les nombres (1 à 24) de même que les cercles, correspondant au site d'insertion du transposon dans pILL753; les signes (+) indiquent que le transposon n'a pas inactivé l'expression de l'uréase alors que les signes (-) indiquent que l'expression de l'uréase a été abolie.

C : Carte de restriction linéaire des plasmides hybrides pILL763 et pILL768 générés par délétion (Δ) à l'intérieur de pILL753. La localisation des gènes (ureA à ureH) est indiquée par des rectangles. La longueur des rectangles correspond à la longueur de l'ADN requis pour exprimer les polypeptides. Les flèches se réfèrent à l'orientation de la transcription. Le nombre de cadres en bas de la figure indique la taille en kilobases des fragments de restriction. Les nombres entre parenthèse correspondent à la taille des fragments d'ADN de H. pylori insérés dans un des vecteurs de clonage (pILL575, pILL550 ou pILL570). B, BamHI; E, EcoRI; P, PstI, H, HindIII; C, ClaI; Sm, SmaI. Les lettres entre parenthèse indiquent que les sites de restriction appartiennent au vecteur.

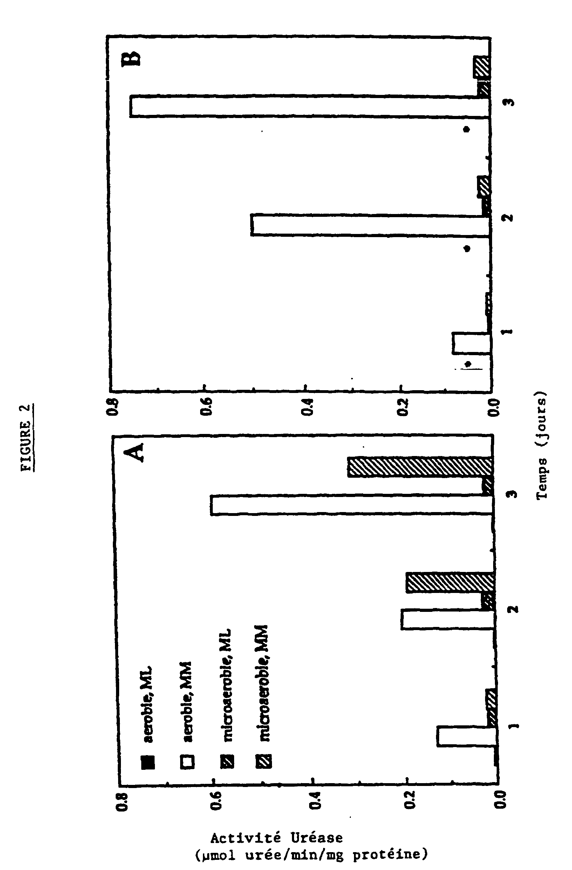

Figure 2: **Activité uréasique exprimée par** E.coli **HB101 hébergeant pILL753, en fonction du temps**

Des boîtes préparées avec soit un milieu L-agar (ML) soit un milieu minimum M9 complétées avec 10 mM L-arginine (MM) ont été chacune inoculées avec une partie aliquote de 100 μl de culture et mises en suspension ($10^8$ bactérie/ml) dans du NaCl stérile, à 0,85%. Les boites ont été incubées, en milieu aérobie ou microaérobie, à (A) 30°C ou (B) 37°C et les mesures de l'activité ont été faites en temps voulu. Les astérisques indiquent qu'aucune activité uréasique n'a été détectée.

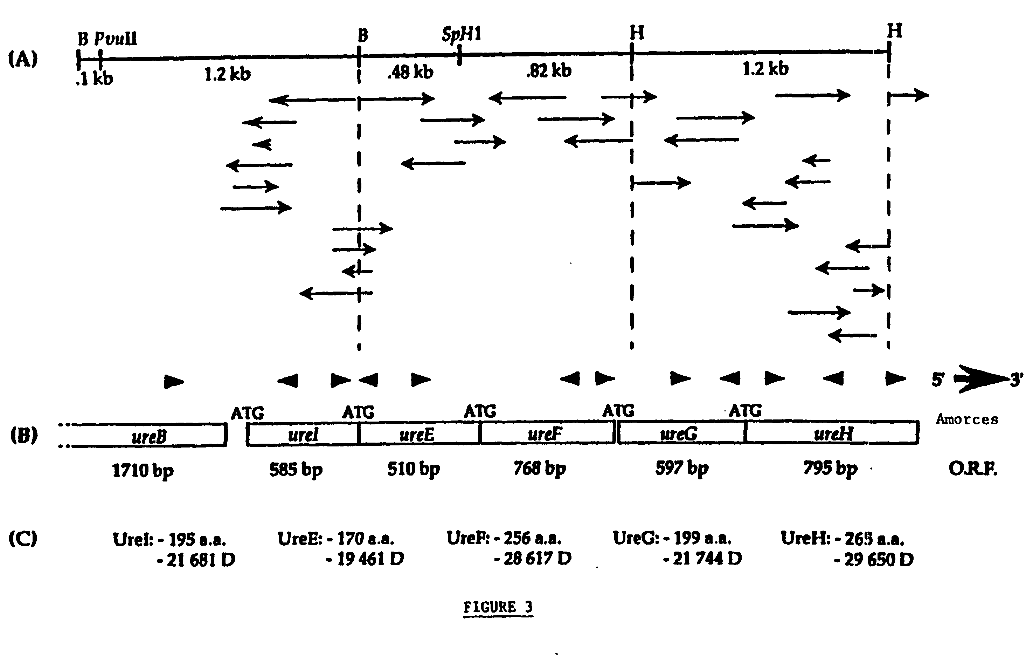

Figure 3: **Séquence d'ADN des gènes accessoires de l'uréase de** H. pylori

A : Stratégie pour le séquençage des gènes accessoires de la région uréase du plasmide hybride pILL753. Les flèches correspondent aux tailles des fragments d'ADN séquencés. Les têtes de flèches représentent les oligonucléotides utilisés pour réaliser et confirmer la détermination oligonucléotidique.
B : Représentation schématique des cinq cadres ouverts de lecture (ORFs) déduits à partir de l'analyse de la séquence nucléotidique et leurs tailles en nucléotides. ATG correspond au codon d'initiation relatif à chacun des gènes.
C : Les tailles et les masses moléculaires calculées des cinq polypeptides supplémentaires de l'uréase de H. pylori sont indiquées.

Figure 4: **Séquence nucléotidique des gènes accessoires de l'uréase de** H. pylori

Les nombres en haut de la séquence indiquent la position des nucléotides. Les séquences d'acides aminés prédites, dans l'ordre séquentiel sont : UreI (bp 211 à 795), UreE (bp 800 à 1309), UreF (bp 1324 à 2091), UreG (bp 2123 à 2719), et UreH (bp 2722 à 3516). Les séquences potentielles de liaison aux ribosomes (Shine-Dalgarno, sites SD), sont soulignées. Les séquences encadrées correspondent aux séquences de type promoteur-like (σ54) et les flèches au dessus de la séquence indiquent les structures en boucle avec les éléments d'un signal de fin de transcription rho-indépendant (Rosenberg et al (1979) Annu. Rev. Genet. 13: 319-359). Les pointillés sous la séquence d'acides aminés correspondent au domaine de liaison de l'ADN (ureI) ou de l'ATP de la protéine (ureG) (Higgins et al (1985) EMBO J. 4: 1033-1040 et Pabo et al (1984) Ann. Rev. Biochem. 53: 293-321).

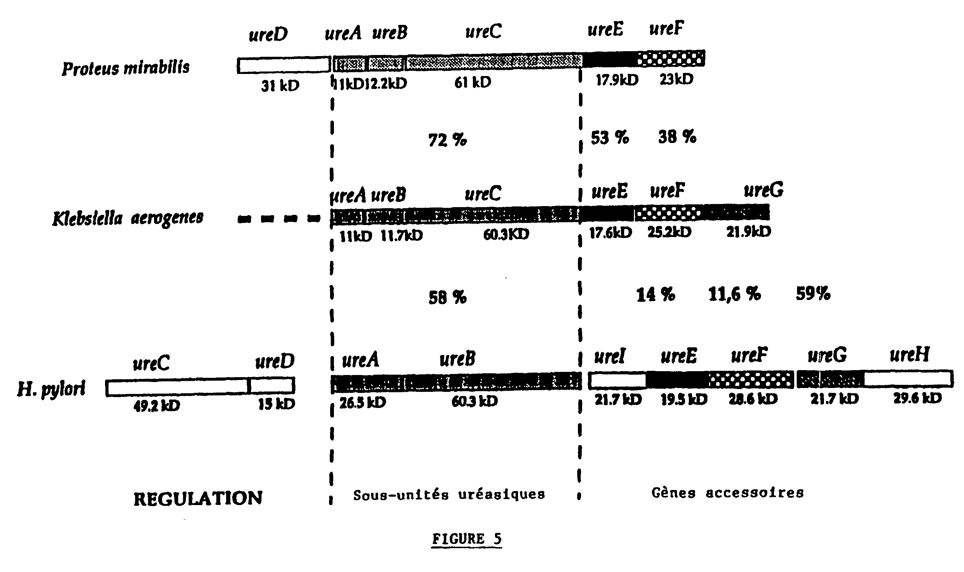

Figure 5: **Organisation génétique de l'opéron uréase**

Les positions relatives des gènes codant pour des polypeptides associés avec l'opéron uréase de P. mirabilis (Jones et al (1989) J. Bacteriol. 171: 6414-6422), de K. aerogenes (Mulrooney et al - 1990) et de H. pylori sont indiquées. Les pourcentages se rapportent à la proportion d'acides aminés identiques entre deux gènes apparentés. Les cadres blancs représentent les gènes qui sont uniques à l'opéron.

Figures 6 et 7 **Analyse des souches parentales et mutées**

Figure 8: **Profils de restriction après digestion enzymatique des ADN totaux des souches 85P, N6 et N6 mutée (uréase⁻)**

Figures 9 et 10 **Organisation génomique des 4 gènes** ure **dans les génomes des souches 85P et N6. Les fragments spécifiques d'ADN ont été amplifiés à partir de l'ADN chromosomique extrait des isolats 85P et N6 de** H. pylori **en utilisant 8 paires d'amorces conformément à la figure 10. Les produits d'amplification ont été séparés par électrophorèse sur un gel d'agarose à 1,4%. Les valeurs de chaque côté du gel correspondent aux dimensions (en kilobases) de l'échelle de 1 kb utilisée comme standard.**

Figure 11 **Immunobuvardage à l'aide d'anticorps**

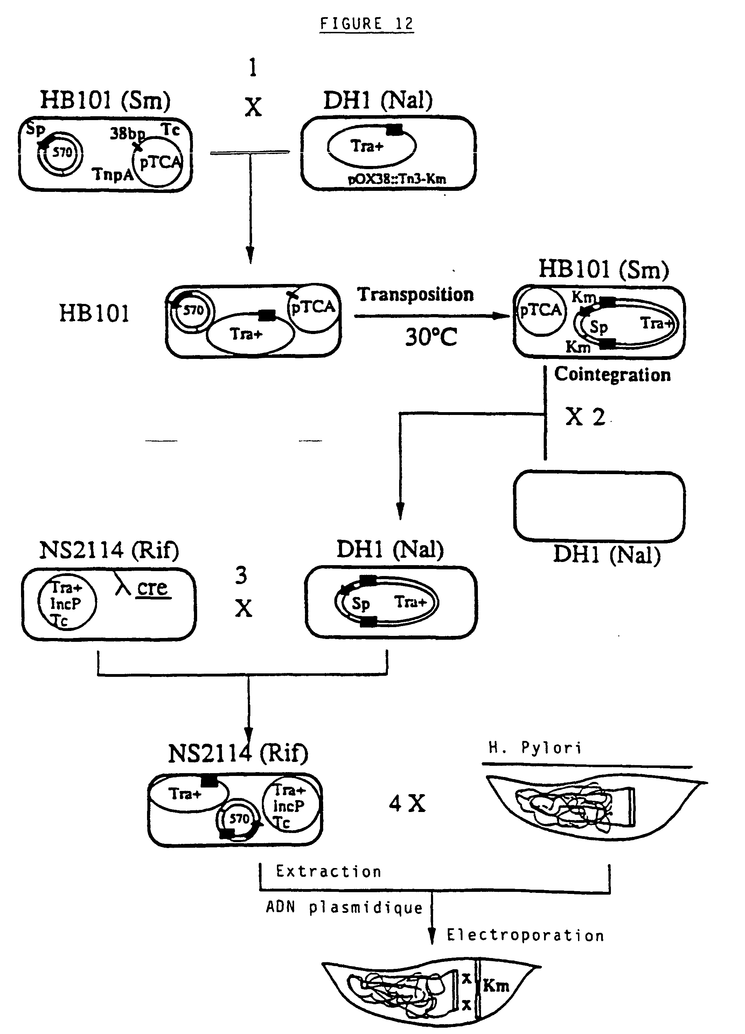

Figure 12 **Mutagénèse par transposon : Représentation schématique de quatre étapes consécutives nécessaires pour la construction de mutants dans une bactérie** H. pylori

Conjugaison 1 : le plasmide transférable pOX38 du groupe IncF hébergeant le transposon MiniTn3-Km est introduit dans E.coli HB 101 contenant 1) le plasmide pTCA exprimant de façon constitutive la transposase Tn3 (TnpA) et immun à Tn3 compte tenu de la présence de la séquence Tn3-38bp et 2) le vecteur suicide de conjugaison contenant le fragment clone de H. pylori à mutagénéiser. Les transconjugants HB101 kanamycine sont cultivés pendant 48 heures à 30°C et les bactéries sont conjuguées avec E.coli DH1 (Nal).

Conjugaison 2 : les cointégrats résultant de la transposition de MiniTn3-Km dans le plasmide dérivé de pILL570 en l'absence de résolvase sont sélectionnés comme cointégrats kanamycine conjugatifs dans les cellules DHl.

Conjugaison 3 : les cointégrats sont introduits dans la souche NS2114 (Rif) hébergeant le gène cre, capable de produire une résolution par recombinaison spécifique du cointégrat en deux réplicons, l'un consistant dans le donneur d'origine pour le transposon (pOX38-MiniTn3-Km) et l'autre consistant dans le plasmide hydride dérivant de pILL570 dans lequel MiniTn3-Km a été inséré. La sélection positive des formes résolues des cointégrats a été obtenue par sélection des transconjugants NS2114 à la kanamycine, sur un milieu contenant 300 μg/ml de kanamycine ainsi que 300 μg/ml de spectinomycine. La dernière étape consistant en l'introduction de l'ADN muté chez H. pylori peut être réalisée en électroporant H. pylori avec l'ADN plasmidique extrait d'E.coli NS2114 (souche de référence), obtenu à l'étape 3.

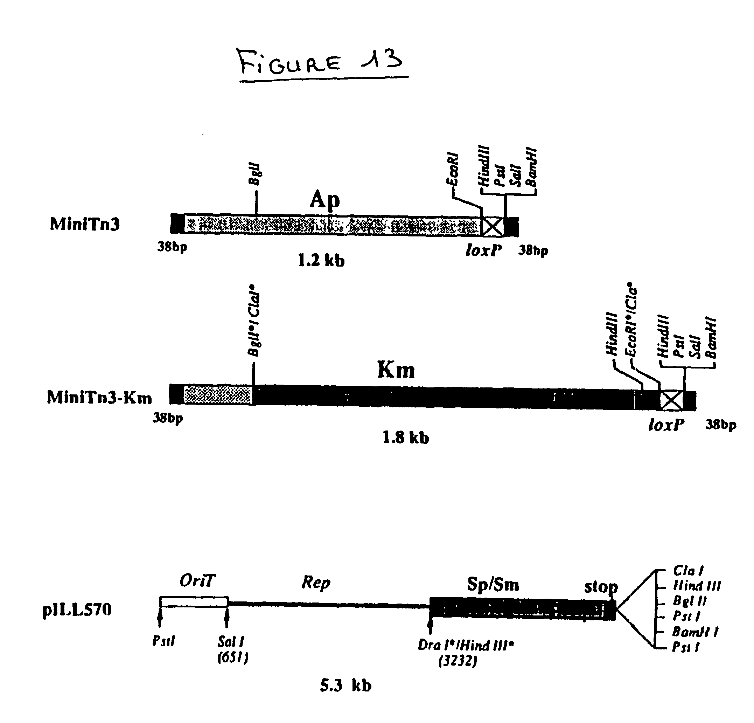

Figure 13 **Carte de restriction de MiniTn3 selon Seifert et al (1986 PNAS, USA, 83:735-739).**

L'étoile indique dans le plasmide pILL570 les sites de restriction qui ont été modifiés au cours de la construction du vecteur.

## I - IDENTIFICATION DES GENES

## MATERIELS ET METHODES

Souches bactériennes, plasmides et conditions de culture

H. pylori 85P a été isolé chez un patient atteint de gastrite, et correspond à la souche décrite dans Labigne et al (J. Bacteriol. 173: 1920-1931 (1991)). E.coli MC1061 (Maniatis et al (1983), Molecular cloning a laboratory manual. Cold Spring Harbor Laboratory, Cold Spring Harbor N.Y.) a été utilisé comme hôte dans les expériences de clonage et E.coli HB101 (HsdR hsdM reA supE44 lacZ4 LeuB6 proA2 thi-1 Sm) (Boyer et al (1969) J. Mol. Biol. 41: 459-472) a été utilisé comme hôte pour l'analyse quantitative de l'expression de l'uréase. Les vecteurs et hybrides utilisés dans cette étude figurent dans le tableau 1. Les souches d'E.coli ont été cultivées dans du bouillon L sans glucose (10 g de tryptone, 5 g d'extrait de levure et 5 g de NaCl par litre, pH = 7,0) ou sur des boites de gélose L (contenant 1,5% de gélose) à 37°C. Les concentrations en antibiotiques pour la sélection des transformants ont été les suivantes (en milligrammes par litre) : kanamycine: 20, tétracycline: 8, ampicilline: 100, spectinomycine: 100, carbénicilline: 100. Pour l'expression de l'activité uréasique, les bactéries E.coli ont été cultivées sur un milieu limitant la concentration en source d'azote constitué de milieu gélosé minimum M9 sans ammonium (pH = 7,4) contenant 0,4% de D-glucose comme source de carbone et, sauf indication contraire, 0,2% (p/v) de L-glutamine stérilisée par filtration et fraîchement préparée (Pahel et al (1982) J. Bacteriol. 150: 202-213) comme source d'azote.

Clonage moléculaire et analyses de l'ADN

Les digestions avec une endonucléase de restriction, le remplissage des extrémités et les autres manipulations courantes de l'ADN ont été effectués selon les techniques standard de Maniatis et coll. (Maniatis et al (1983), Molecular cloning a laboratory manual. Cold Spring Harbor Laboratory, Cold Spring Harbor N.Y.). Les digestions partielles avec Sau3A ont été faites à 20°C de façon à ralentir l'activité enzymatique. Les endonucléases de restriction, le grand fragment de l'ADN polymérase I, l'ADN polymérase de T4 (utilisée pour rendre franches les extrémités des fragments) et l'ADN ligase de T4 ont été fournis par Amersham Corp. La phosphatase alcaline d'intestin de veau a été fournie par Pharmacia. Les fragments d'ADN ont été séparés par électrophorèse sur des blocs horizontaux de gel contenant 1 ou 1,4% d'agarose et traités dans des tampons Tris-acétate ou Tris-phosphate (Maniatis et al (1983), Molecular cloning a laboratory manual. Cold Spring Harbor Laboratory, Cold Spring Harbor N.Y.). Une échelle de 1 kb (Bethesda Research Laboratories) a été utilisée comme standard de poids moléculaire. L'électroélution des fragments d'ADN à partir des gels d'agarose contenant du bromure d'éthidium (0,4 μg/ml) a été effectuée comme précédemment décrit (J. Bacteriol. 173: 1920-1931 (1991), Labigne et al).

Activité uréasique

La détection de l'activité uréasique a été effectuée par remise en suspension de $10^9$ bactéries dans 1 ml de milieu urée-indole (Diagnostic Pasteur) et incubation à 37°C pendant des périodes variables. La libération de l'ammoniac due à l'activité uréasique a élevé le pH en provoquant un virage de l'orange au rouge.

L'activité uréasique a été mesurée selon la réaction de Berthelot, selon une modification du mode opératoire précédemment décrit (Ferrero et al (1991) Microb. Ecol. Hlth. Dis. 4: 121-134). Succinctement, les bactéries ont été recueillies à partir de boîtes de gélose dans 2,0 ml de NaCl à 0,85% stérile et centrifugées à 12 000 tr/min pendant 10 minutes à 4°C. Les culots ont été lavés deux fois dans du NaCl à 0,85% et remis en suspension dans du tampon phosphate de sodium à 100 mM (pH 7,4) contenant 10 mM d'EDTA (PEB). Pour préparer les extraits traités aux ultrasons, les cellules ont été lysées par quatre impulsions de 30 s avec un Branson Sonifier Model 450 réglé à 30 W, cycle 50%. Les débris cellulaires ont été éliminés avant les déterminations de l'uréase. Les échantillons fraîchement préparés (10-50 µl) ont été ajoutés à 200 µl de solution substrat d'urée (urée 50 mM préparée dans le PEB) et mis à réagir à la température ordinaire pendant 30 minutes. Les réactions ont été arrêtées par addition de 400 µl de réactif de phénol-nitroprussiate et 400 µl de réactif hypochlorite alcalin. Le mélange réactionnel a été incubé à 50°C. Des blancs, dans lesquels l'activité uréasique était inactivée par l'ébullition pendant 5 minutes avant l'addition du substrat, ont été traités de façon semblable. La quantité d'ammoniac libérée à été déterminée à partir d'une courbe d'étalonnage établissant la relation entre $A_{625}$ et la concentration en ammonium (à partir de $NH_4Cl$). On a considéré que la libération de 2 µmol d'ammoniac équivalait à l'hyrdolyse de 1 µmol d'urée. L'activité uréasique a été exprimée en µmol d'urée hydrolysée/min/mg de protéine bactérienne.

Détermination des protéines

Les concentrations des protéines ont été déterminées selon l'essai de Bradford (Sigma Chemicals). Pour solubiliser les protéines dans les extraits de cellules entières, les suspensions cellulaires préparées dans du TPE ont été centrifugées et les culots ont été remis en suspension dans une solution d'octyl-β-D-glucopyrannoside, afin d'établir une concentration finale en détergent (dans le réactif colorant) de 0,1-0,2% (p/v).

Mutagénèse de transposon et construction de mutants

Le système d'apport MiniTn3-Km a été utilisé pour produire des mutations par insertion aléatoire dans le fragment d'ADN cloné dans pILL570.

Le système MiniTn3 décrit par Seifert et al (1986 PNAS USA 83: 735-739) faisant intervenir le plasmide pOX38 en tant que donneur de l'élément transposable et le plasmide pTCA agisant en trans et apportant l'enzyme transposase Tn3 (Seifert et al 1985 Genetic Engineering Principles and Methods Vol. 8: p.123-134 Setlow, J. and Hollaeinder, A. Editors, Plenum Press New-York), et la souche NS2114 hébergeant le gène cre codant pour la recombinase P1 spécifique pour le site lox ont été utilisés pour la mutagénèse de fragments d'ADN avec les modifications suivantes :

i) le MiniTn3 a été modifié en enlevant le fragment BgII-EcoRI du gène codant pour la β-lactamase dans le plasmide pTn (Seifert et al 1986 précité), et en le remplaçant par la cassette kanamycine ClaI-C. jejuni (1,4 kb de longueur décrit par Labigne-Roussel et al (1988 J. Bacteriol. 170: 1704-1708)). Ce nouvel agent d'insertion MiniTn3-Km a été transposé dans le plasmide transférable pOX38 tel que décrit par Seifert et al (1986 précité) conduisant à l'obtention du plasmide pILL553;

ii) on a utilisé le vecteur suicide pILL570 spectinomycine conjugatif préalablement décrit par Labigne et al (1991 J. Bacteriol. 173: 1920-1931) pour le clonage du fragment utilisé pour la mutagénèse. Ce vecteur suicide était dérivé de pILL560 (Labigne-Roussel et al 1988 J. Bacteriol. 170: 1704-1708) dont les séquences d'ADN responsables de l'immunité à Tn3 ont été délétées;

iii) le plasmide IncP, pRK212.1 du "complementing plasmid" (Figurski et al 1979 PNAS USA 76: 1648-1652) a été introduit par conjugaison dans E.coli souche NS2114 et un mutant rifampicine spontané de NS2214 hébergeant le gène cre a été obtenu et utilisé pour la sélection des transconjugants hébergeant le cointégrat;

iv) la résolution efficace des cointégrats (produits de co-intégration) a été sélectionnée de manière positive grâce au grand nombre de copies du plasmide dérivé de pILL570 en déposant sur des bôites le troisième mélange obtenu sur un milieu contenant 500 µg de kanamycine et 300 µg de spectinomycine.

Séquençage de l'ADN

Les fragments appropriés d'ADN ont été clonés dans M13mp19 et M13mp18 (Meissing et al (1982) Gene 19: 269-276) pour lire indépendamment les deux brins complémentaires. Les clones contenant les fragments d'insertion ont été identifiés à l'aide de X-Gal (5-bromo-4-chloro-3-indolyl-β-D-galactopyrannoside) et d'isopropyl-β-D-thiogalactopyrannoside. Les brins simples des plasmides recombinés M13mp18 et M13mp19 ont été obtenus selon la méthode au polyéthylèneglycol (Sanger et al (1980) J. Mol. Biol. 143: 161-178). Le séquençage a été effectué selon la méthode d'arrêt de chaîne avec des didésoxynucléotides (Sanger et al (1977) Proc. Natl. Acad. Sci. USA 74: 5463-5467) à l'aide du nécessaire Sequenase (United States Biochemical corp.). Le séquençage de l'ADN bicaténaire a également été effectué par arrêt de chaîne avec des didésoxynucléotides avec le nécessaire Sequenase par emploi d'ADN plasmidique purifié sur un gradient de chlorure de césium (Zhang et al (1988) Nucleic Acids Research. 16: 1220). Des échantillons de 3 μg d'ADN ont été d'abord dénaturés avec une solution de NaOH 1 M (volume total 20 μl), puis neutralisés avec 2 μl d'acétate d'ammonium 2 M (pH 4,6). L'ADN a été précipité après addition de 60 μl d'éthanol à 100% glacé, incubation à -70°C pendant 10 Minutes et centrifugation à 4°C pendant 20 minutes. Après lavage avec 60 μl d'éthanol à 80% glacé, le culot a été remis en suspension dans 10 μl de tampon de séquençage contenant 0,5 pmol de l'amorce et incubé pendant 3 minutes à 65°C. Après une période d'incubation de 30 minutes à la température ordinaire, le séquençage a été effectué.

## RESULTATS

### Détection de l'activité uréasique dans une souche hôte d'E.coli hébergeant le cosmide recombinant pILL585

Des transformants d'E.coli hébergeant le cosmide pILL585 ont été étalés sur du milieu minimum M9 glucosé additionné de 0,2% de 1-glutamine (comme seule source d'azote) ou du milieu L, et incubés à 37°C pendant 48 heures. Les transformants ont été ensuite criblés relativement à l'activité uréasique selon un essai colorimétrique qualitatif effectué dans du milieu urée-indole. L'activité n'a été observée que dans les transformants de E.coli HB101 ayant subi plusieurs passages (plus de 5 passages) sur le milieu minimum à 37°C en conditions d'aérobie. Ce sont donc les conditions qui ont été utilisées pour la détermination qualitative de l'expression de l'uréase dans les clones d'E.coli. Aucune activité uréasique n'a été détectée dans les transformants cultivés sur un milieu riche en azote.

La transformation de la souche E.coli HB101 avec le plasmide pILL590 contenant un fragment de 4,2 kb, identifié comme la région minimale nécessaire à l'expression de l'uréase dans C. jejuni (Labigne et al (1991) J. Bacteriol. 173: 1920-1931) dans les cellules d'E.coli, même après culture et passages dans un milieu limitant la concentration en source d'azote. Cela implique que les gènes présents sur le cosmide, mais absents du plasmide pILL590, sont nécessaires à l'expression de l'uréase chez E.coli.

### Sous-clonage des gènes nécessaires à l'activité uréasique dans une souche d'E.coli

En l'absence d'activité uréasique détectable dans la souche d'E.coli hébergeant le plasmide recombinant pILL590, le fragment d'insertion de 34 kb du cosmide pILL585 a été soumis à une digestion partielle avec l'endonucléase Sau3A pour produire des fragments compris entre 7 et 12 kb. Ils ont été traités à la phosphatase alcaline, pour éviter tout réarrangement du génome initial, et ligaturés au plasmide pILL570 linéarisé avec BamHI. Après transformation dans E.coli HB101, chaque transformant résistant à la spectinomycine a été soumis à un essai ultérieur de sa capacité à hydrolyser l'uréase dans des conditions d'induction. Un clone présentait un phénotype uréase-positif. Il hébergeait un plasmide recombinant appelé pILL753. Ce plasmide contenait un fragment d'insertion de 11,2 kb. Les sites de reconnaissance BamHI et HindIII ont été cartographiés relativement aux sites de restriction uniques EcoR1 et PstI du vecteur pILL570 (figure 1). La comparaison de la carte de restriction du plasmide pILL753 avec celle du plasmide recombinant précédemment décrit pILL590 a démontré que le fragment d'insertion de pILL753 avait un fragment d'ADN additionnel de 4,6 kb situé en aval des quatre gènes de l'uréase précédemment identifiés dans le plasmide pILL590 (c'est à dire ureA, ureB, ureC et ureD).

### Optimisation de l'activité uréasique dans E.coli HB101

Pour définir les conditions de culture assurant l'expression optimale des gènes de l'uréase de H. pylori dans E.coli, l'activité de clones hébergeant pILL753 a été évaluée quantitativement après culture sur un milieu minimum additionné de diverses sources d'azote. Dans tous les cas, un milieu de base minimum solide a été utilisé, car des études ont montré que l'activité uréasique était très faible, dans les cultures effectuées dans un milieu liquide.

Les activités relatives des cultures sur des milieux complétés par L-arginine, L-glutamine, L-glutamate, NH$_4$Cl et l'urée (chacun à une concentration finale de 10 mM) étaient respectivement : 100%, 36%, 27%, 46% et 20%.

L'activité uréasique a été optimale dans les cultures effectuées sur un milieu additionné de L-arginine. L'activité uréasique n'a pas été détectée dans les cultures effectuées sur un milieu riche en azote.

Bien que la présence d'ions Ni$^{2+}$ libres puisse avoir un effet de stimulation de l'activité uréasique (Mulrooney et al (1989) J. Gen. Microbiol. 135: 1769-1776 et Mobley et al (1989) Microbiol. Rev. 53: 85-108), ceci ne s'est pas manifesté sur l'activité uréasique des cellules hébergeant pILL753.

L'analyse au cours de l'expression de l'uréase dans le clone d'E.coli portant pILL753, cultivé dans diverses conditions, a indiqué que l'activité uréasique maximale était obtenue après 3 jours de culture aérobie à 37°C sur du milieu minimum additionné de L-arginine (figure 2). L'activité uréasique dans les cultures effectuées sur un milieu riche en azote était meilleure après culture en microaérobiose. En revanche, des conditions de microaérobie ont eu un effet répressif sur les activités des cultures limitées en azote.

L'activité uréasique des cellules E.coli hébergeant pILL753 en culture en conditions d'aérobie pendant 3 jours à 37°C dans un milieu minimum additionné avec de l'arginine, était 0,9 ± 0,4 µmol d'urée hydrolyse par minute, par mg de protéine. Par comparaison l'isolat de H. pylori utilisé pour cloner les gènes de l'uréase hydrolysait l'urée à un taux de 23,2 ± 2,3 µmol/mn/mg protéine.

Identification et localisation des gènes nécessaires à l'activité uréasique dans une souche hôte d'E.coli

Pour déterminer la région d'ADN nécessaire au phénotype uréase-positif, des dérivés de pILL753 portant l'élément transposable MiniTn3-Km ont tout d'abord été isolés selon un mode opératoire précédemment décrit (voir Matériels et Méthodes). Des transformants d'E.coli HB101 portant les transposons ont tous été criblés relativement à l'activité uréasique. Ils ont été appelés pILL753::x où x désigne le site d'insertion de MiniTn3-Km tel qu'il apparaît sur la carte de la figure 1. Sur les 24 insertions choisies pour l'analyse, 10 dérivés avaient totalement perdu la capacité d'hydrolyser l'urée (2, 3, 4, 5, 6, 10, 11, 12, 13 et 14), tandis que 14 conservaient le phénotype uréase-positif. Ces résultats confirment que toute mutation d'insertion ayant une cartographie correspondant aux gènes ureA ou ureB (mutants 2, 3, 4, 5 et 6) abolit l'activité uréasique, mais démontrent également qu'un fragment d'ADN de 2,6 kb situé plus en aval du gène ureB est nécessaire à l'expression d'un phénotype uréase-positif dans E.coli cultivé dans des conditions limitant l'azote. En revanche, à partir des résultats relatifs à la mutagénèse des transposons, un fragment d'ADN de 600 pb situé immédiatement en aval du gène ureB ne s'est pas révélé être essentiel à l'expression de l'activité uréasique dans E.coli.

Des analyses additionnelles comprenant l'établissement de délétions dans le fragment d'insertion pILL753 ont été effectuées pour mieux comprendre les conditions nécessaires à l'expression d'une uréase active dans les cellules d'E.coli. Des sous-clones d'E.coli portant les dérivés plasmidiques ont fait l'objet d'une détermination quantitative de l'activité uréasique dans les conditions de limitation de l'azote définies ci-dessus. Les résultats sont résumés au tableau 2. Tous les sous-clones étaient des dérivés du même vecteur pILL570, si bien que les résultats peuvent être comparés. L'un deux, le plasmide pILL768, a été obtenu par autoreligature du grand fragment EcoRI produit à partir du produit de digestion par une enzyme de restriction du plasmide pILL753::16 (figure 1). Cette construction a conduit à une délétion de 2,95 kb à l'extrémité 3' du segment d'insertion pILL753. Les cellules portant ce plasmide expriment une activité uréase comparativement faible (tableau 2). Le plasmide pILL763, a été obtenu par clonage du fragment de restriction ClaI-PstI du plasmide pILL753::1 dans le vecteur pILL570 linéarisé. Cette construction, dans laquelle un fragment d'ADN de 1,75 kb contenant les gènes ureC et ureD, précédemment décrits, était supprimé, exprimait une activité uréasique approximativement deux fois plus fortes que celles des cellules hébergeant pILL753. Dans aucun cas, des délétions ou des insertions n'ont conduit à une activité uréasique constitutive.

Analyse de la séquence de la région nécessaire à l'expression de l'uréase dans E.coli

Dans le fragment de 11,2 kb nécessaire à l'expression de l'uréase dans E.coli, un fragment d'ADN de 3,2 kb localisé immédiatement en aval du gène ureB, a été identifié suivant la stratégie de la figure 3.

i) les fragments HindIII de 1,2 kb et BamHI-HindIII de 1,3 kb, ont été séquences indépendamment après : a) clonage des fragments de restriction précités, b) des fragments SpHI-BamHI, SpHI-HindIII, c) des fragments BamRI-HindIII des plasmides pILL753::12, pILL753::11; pILL753::10 dans l'ADN des phages M13mp18 et M13mp19;

ii) les fragments de restriction HindIII de 1,2 kb, BamHI-PstI de 3,8 kb et BamHI-PvuII de 1,3 kb provenant des plasmides pILL753 et pILL589 (précédemment décrits) ont été clonés dans l'ADN des phages M13mp18 et M13mp19;

iii) douze amorces oligonucléotidiques ont été synthétisées pour confirmer la lecture et produire des séquences chevauchant les trois fragments séquencés indépendamment. Ces amorces ont été utilisées pour des analyses de séquençage d'ADN double brin.

L'analyse de la séquence a révélé cinq cadres de lecture ouverts (ORFs) appelés ureI, ureE, ureF, ureG et ureH. Ces gènes sont tous transcrits dans la même direction et il est prévu qu'ils codent pour des peptides de 195, 170, 256, 199 et 265 acides aminés. Aucun ORF de longueur notable n'a été observé sur le complément inverse de la séquence illustrée par la figure 4. Les cinq ORF débutent par le codon de départ caractéristique ATG. Quatre des cinq ORF étaient précédés de sites semblables à la séquence consensus de liaison au ribosome (Shine-Dalgarno) d'E.coli (Shine et al (1974) Proc. Natl. Acad. Sci. USA 71: 1342-1346).

Les régions d'amont de chaque ORF ont fait l'objet d'une recherche de la présence des sites de régulation azotée avec la séquence $TGGYAYRN_4YYGCZ$ dans laquelle Y = T ou C, R = G ou A et Z = A ou T (Morett et al (1989) J. Mol. Biol. 210: 65-77). Un seul site a été trouvé à 210 pb en amont du locus ure G. Sa position précise est représentée sur la figure 4. Des séquences consensus de type promoteur d'E.coli (σ70) ont été observées en amont des gènes ureI, ureF et ureH (TTGACA, -35 et TATAAT, -10).

**TABLEAU 1 :**

Vecteurs et plasmides hybrides utilisés dans le cadre de cette étude

| Plasmide | Vecteur | Caractéristiques * phénotypiques | Taille (kb) | Origine de l'insertion | Références |
|---|---|---|---|---|---|
| | pILL550 | RepEcRepCj mob Km | 8,3 | | Labigne-Roussel et al |
| | pILL570 | RepEcmob Sp | 5,3 | | Labigne A. et al |
| | pILL575 | RepEcRepCj mob Km Cos | 10 | | Labigne A. et al |
| pILL585 | pILL575 | RepEcRepCj mob Km Cos | 44 | Sau3A digestion partielle de 85P | Labigne A. et al |
| PILL590 | pILL550 | RepEcRepCj mob Km | 16,4 | Sau3A digestion partielle de pILL585 | Labigne A. et al |
| pILL753 | pILL570 | RepEcmob Sp | 16,5 | Sau3A digestion partielle de pILL585 | décrit ici |
| pILL763 | pILL570 | RepEcmob Sp | 14,75 | Fragment ClaI-PstI de pILL753::1 | décrit ici |
| pILL768 | pILL570 | RepEcmob Sp | 15,35 | Fragment EcoR1 de pILL753::16 | décrit ici |

* RepEc et RepCj : plasmides capables de se répliquer dans E.coli et C. jejuni respectivement -
mob : plasmide mobilisable du à la présence de Ori-T
Km et Sp : résistance à la kanamycine et à la spectinomycine
Cos : présence d'un site cos

TABLEAU 2 :

| Plasmide | Génotype différent de E.coliHB101 pILL753 | Valeurs moyennes Activité uréase (µmol urea/min/mg) |
|---|---|---|
| **Mutagénèse de l'ADN cloné de H. pylori et effet sur l'activité uréasique dans les clones de E.coli HB101 mis en culture dans des conditions limitantes en asote** | | |
| pILL753 (2) | - | 0,86 ± 0,39 |
| pILL753::3 | ureA dégradée | neg (3) |
| pILL753::6 | ureB dégradée | neg |
| pILL753::8 | ureI dégradée | 1,1 ± 0,23 |
| pILL753::10 | ureF dégradée | neg |
| pILL753::11 | ureG dégradée | neg |
| pILL753::13 | ureH dégradée | neg |
| pILL753::16 | insertion en aval ureH | 0,66 ± 0,11(4) |
| pILL763 | ureC et ureD délétées | 2,14 ± 0,16 |
| pILL768 | délétion en 3' en aval de ureH | 0,57 ± 0,28 |

(1) Bactéries mises en culture dans un milieu aérobie pendant 3 jours sur milieu minimum M9 complété avec 0.01 M L-arginine à 37°C.

(2) Pour comparaison, l'activité uréasique de H.pylori 85P, isolat à partir duquel l'ADN a été cloné, était de 23 ± 2.3 µmol urea/min/mg protéine.

(3) Aucune activité uréasique n'a été détectée.

(4) Résultat pour une mesure particulière : 0,73 (5) Résultat pour une mesure particulière : 0,10

**DISCUSSION**

Le premier cas d'expression fonctionnelle de gènes provenant de H. pylori dans des souches d'E.coli est presente ici.

Ceci a été possible en cultivant des cellules de E.coli hébergeant le cosmide recombinant de l'uréase pILL585 (Labigne et al précité - 1991) sur un milieu minimum contenant une source limitant l'azote. Les résultats obtenus ont permis de montrer que les gènes de l'uréase de H. pylori pouvaient être sous le contrôle du système de régulation de l'azote (NTR); et que l'activité uréasique dans les cellules de E.coli était dépendante de la présence d'un ensemble de gènes qui ont été décrits dans les pages précédentes. Cet ensemble de gènes a été localisé immédiatement en aval des quatre gènes ureA, ureB, ureC et ureD décrits dans la publication de Labigne et al, 1991 précitée. Ces nouveaux gènes sont situés sur un fragment de 3,2 kb comportant cinq cadres ouverts de lecture qui sont désignés par ureI, ureE, ureF, ureG et ureH.

La mise en oeuvre de mutations insertionnelles, et de délétions, au niveau du fragment d'ADN de 11,2 kb (pILL753) sous-cloné à partir du cosmide d'origine, a permis de montrer que les gènes ureA, ureB, ureF, ureG et ureH étaient nécessaires à l'expression de l'activité uréasique dans E.coli. Au contraire des mutations insertionnelles à l'intérieur du gène ureI n'ont pas affecté sensiblement l'activité uréasique dans les cellules de E.coli. La délétion du gène ureC et du gène ureD (comme dans le plasmide pILL763) a résulté dans des activités qui étaient significativement plus fortes que celles obtenues dans les cellules portant les plasmides avec les loci intacts, suggérant un rôle régulateur de cette région du cluster du gène de l'uréase de H. pylori.

Il apparaît clair que pILL753 ne porte probablement pas l'ensemble des éléments nécessaires à l'expression complète de l'uréase. La principale preuve pour cela est que : d'une part les cellules de E.coli hébergeant pILL753 avaient une activité uréasique approximativement 25 fois plus faible que celle de l'isolat H. pylori utilisé à l'origine pour le clonage; d'autre part la délétion de la région en aval de ureH (pILL768) a conduit à une diminution considérable de l'activité uréasique. Il est intéressant de remarquer que C. jejuni nécessite la présence d'un nombre de gènes plus faible pour l'expression enzymatique en comparaison avec les résultats obtenus chez E.coli. En conséquence C. jejuni doit être capable de complémenter les fonctions des gènes clonés de H. pylori.

La nécessité de gènes accessoires a également été démontrée pour les Providencia stuartii (Mulrooney et al (1988) J. Bacteriol. 170: 2202-2207), un E.coli uréase positif (Collins et al - 1988), Klesiella pneumonia (Gerlach et al (1988) FEMS Microbiol. Lett. 50: 131-135), Proteus vulgaris (Mörsdorf et al (1990) FEMS Microbiol. Lett. 66: 67-74), Staphylococcus saprophyticus (Gatermann et al (1989) Infect. Immun. 57: 2998-3002), Klebsiella aerogenes (Mulrooney et al - 1990) et Proteus mirabilis (Jones et al (1989) J. Bact. 171: 6414-6422 et Walz et al (1988) J. Bacteriol. 170: 1027-1033).

La figure 5 présente une comparaison de trois régions codant pour l'uréase, pour plusieurs espèces de bactéries et montre les similitudes ainsi que les particularités de chacune. Le degré de parenté, en terme d'organisation génétique et de polypeptides codés, est plus fort entre P. mirabilis et K. aerogenes que pour chacun d'entre eux vis à vis de H. pylori. Alors que le polypeptide UreG de H. pylori présentait une similitude forte avec celui de K. aerogenes (92% de conservation et 59% d'identité), les degrés de (conservation et identité) entre les polypeptides UreE et UreF de H. pylori et K. aerogenes étaient : (33% et 14%), (44% et 11,6%), respectivement. Mulrooney et al ont constaté que les gènes de K. aerogenes codant pour les protéines accessoires UreE, UreI et UreG sont impliqués dans l'activation de l'apoenzyme par incorporation de nickel dans des sous-unités de l'uréase. Du fait de la présence de séries de résidus hystidine à l'extrémité carboxy-terminale du polypeptide UreE de Klebsiella et de Proteus, Mulrooney at al ont proposé que UreE pourrait interagir avec le nickel pour le transférer ensuite à l'apoenzyme. Une telle série de résidus n'a pas été trouvée sur le polypeptide UreE de H. pylori ni sur aucun autre des produits des gènes uréases.

La recherche de similitudes entre la séquence d'acides aminés déduite des gènes de l'uréase de H. pylori et les séquences consensus impliquées dans un site de liaison de l'ADN (Pabo et al - 1981) ou dans des sites de liaison de l'ATP (Higgins et al - 1985) a permis l'identification d'un site de liaison de l'ADH à l'intérieur du produit du gène ureI (figure 4). De plus un site de liaison de l'ATP bien conservé (-GVCGSGKT-) existe à l'extrémité NH2-terminale du produit du gène ureG.

La région de l'uréase de H. pylori présente certains éléments uniques qui sont les suivants : tout d'abord les gènes ureC, ureD, ureI sont uniques pour H. pylori. Ensuite la région de l'uréase consiste en trois blocs de gènes qui sont transcrits dans la même direction et présentent une région intergénique de 420 bp entre ureD et ureA et 200 bp entre ureB et ureI. Ceci suggère une organisation génétique particulière à H. pylori, dans laquelle les trois blocs de gènes peuvent être régulés indépendamment.

Il est généralement admis que la synthèse d'uréase par H. pylori se fait de façon constitutive. Les résultats présentés ici tendent à montrer que l'expression des gènes de l'uréase de H. pylori pourrait en fait être sous le contrôle d'un système de régulation. En effet l'expression des gènes uréases de H. pylori une fois transférés chez E.coli, est totalement sous le contrôle du système de régulation de l'azote (NTR). Il est possible que les gènes de l'uréase de H. pylori, soient directement dépendants de la synthèse des produits des gènes ntrA, ntrB, ntrC de E.coli mais on ne peut exclure qu'ils soient dépendants de l'expression d'un ou de plusieurs autres gènes codant pour une (des) protéine (s) régulatrice (s) analogue (s) aux produits ntr de E.coli. Sur la base de ces données on peut penser que des paramètres physiologiques, tels que la présence d'un milieu solide ou d'une atmosphère microaérophile puisse jouer un rôle dans l'expression de l'uréase chez H. pylori in vitro ou in vivo.

## II - PREPARATION DE SOUCHES MUTANTES

- Souches utilisées pour les expériences d'électroporation : plusieurs souches isolées de biopsies ont été testées pour leur aptitude à être électroporées incluant la souche 85P décrite dans la publication de Labigne et al - 1991 précitée, à partir de laquelle le clonage initial des gènes de l'uréase a été réalisé. Une seule souche désignée N6 déposée à la CNCM sous le numéro I-1150 le 3 Octobre 1991, a donné des résultats positifs.

- Création des mutants dans le fragment cloné du chromosome de H. pylori, souche 85P : les mutants sont préparés par mutagénèse à l'aide d'un transposon (MiniTn3-Km) qui permet l'insertion au hasard de l'élément (élément de transposition). Le site d'insertion de chacun des éléments de transposition a été défini par analyses de restriction des plasmides dérivés (cf figure 1).

- Electroporation : $10^{10}$ cellules de H. pylori ont été récoltées sur gélose au sang (10% de sang de cheval) lavées dans une solution de glycérol/sucrose (15% v/v et 9% w/v) et resuspendues sous un volume de 50 µl à 4°C. 500 ng d'ADN plasmidique purifié sur CsCl et dialysé extemporanément contre de l'eau distillée ont été ajoutés sous un volume de 1 µl aux cellules à 4°C. Après 1 minute sur la glace les cellules d'ADN ont été transférées dans une cuvette d'électroporation prérefroidie à -20°C (BioRad réf : 165-2086, de 0,2 cm de largeur), puis placées dans l'appareil "Gene pulser Apparatus - BioRad) pour lequel il a été fixé les paramètres suivants : 25F, 2.5kV et 200 ohms. Après avoir délivré l'impulsion électrique avec des constantes de temps 4,5 à 5 msec, les bactéries ont été resuspendues dans 100 µl de tampon SOC (2% Bacto tryptone, 0,5% Bacto yeast extract, 10 mM NaCl, 2,5 mM KC1, 10 mM $MgCl_2$, 10 mM MgSO4, 20 mM glucose), et inoculées sur gélose au sang non sélective (sans kanamycine, mais incluant vancomycine, trimethoprime, polymixine, acide nalidixique, amphotéricine B) pendant 48 heures à 37°C sous atmosphère microaérophile. Les bactéries sont ensuite récoltées, resuspendues dans un volume de milieu Brucella (0,5 ml) et 100 µl de la suspension sont étalés sur boites de gélose au sang sélective (incluant 20 µg/ml de kanamycine et le coktail antibiotique décrit ci-dessus). La croissance de bactéries transformées et résistantes à la kanamycine apparaît après 4 jours d'incubation à 37°C en atmosphère microaérophile.

Les autres techniques incluant PCR, Southern, et Western sont des techniques classiques.

## RESULTATS

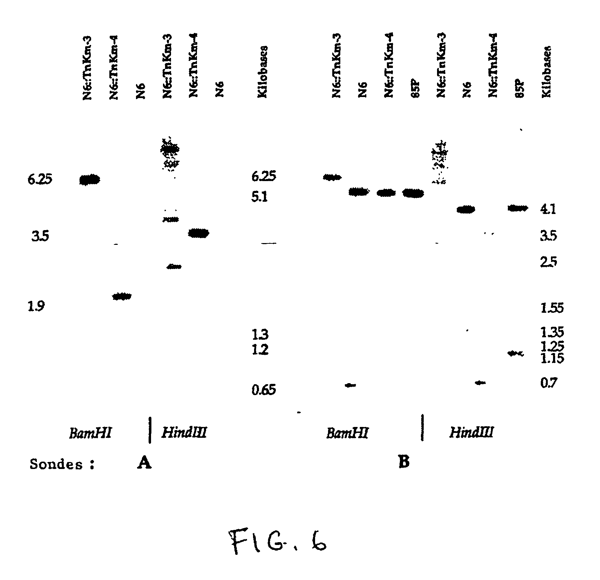

Deux mutations générées par insertion de MiniTn3-Km dans le gène ureB présent sur le plasmide pILL753 ont été étudiées en détail. Il s'agit des mutations numérotées 3 et 4. La position précise de chacune des insertions est donnée à la figure 6. Les plasmides correspondant à ces insertions ont été préparés, purifiés et concentrés. Des bactéries résistantes à la kanamycine présentant toutes les caractéristiques de la souche N6 H. pylori utilisée pour l'électroporation ont été obtenues; elles sont totalement incapables d'hydrolyser l'urée.

Des contrôles ont permis de vérifier que la souche mutante est une souche isogénique :

*   bien que "uréase négative" les souches ont les propriétés biochimiques caractéristiques des bactéries appartenant à l'espèce H. pylori (oxydase, catalase, sensibilité à l'oxygène);

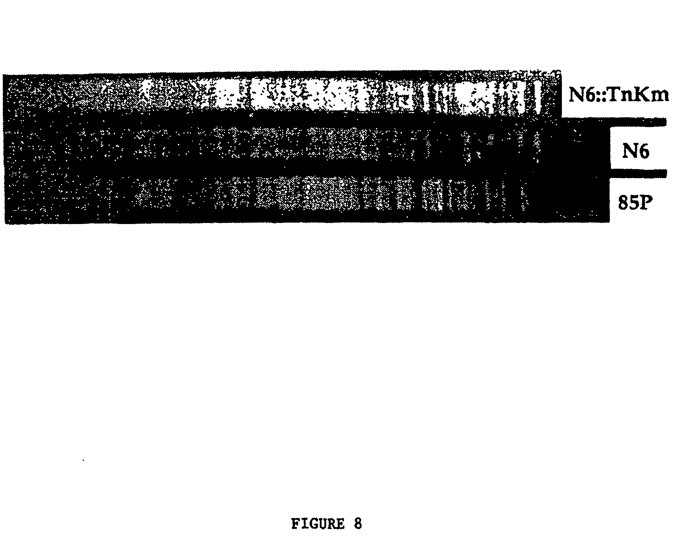

*   les bactéries mères (N6) (CNCM n°I-1150) et les bactéries isogéniques N6::TnKm-3 et N6::TnKm-4 ont les mêmes profils de restriction après digestion enzymatique des ADN totaux (cf figure 8);

*   après amplification enzymatique à l'aide des primers spécifiques de H. pylori et séquençage du produit amplifié, les mêmes séquences nucléotidiques ont été trouvées alors que des souches indépendantes de H. pylori ne présentent jamais la même séquence, mais au contraire un polymorphisme génique important;

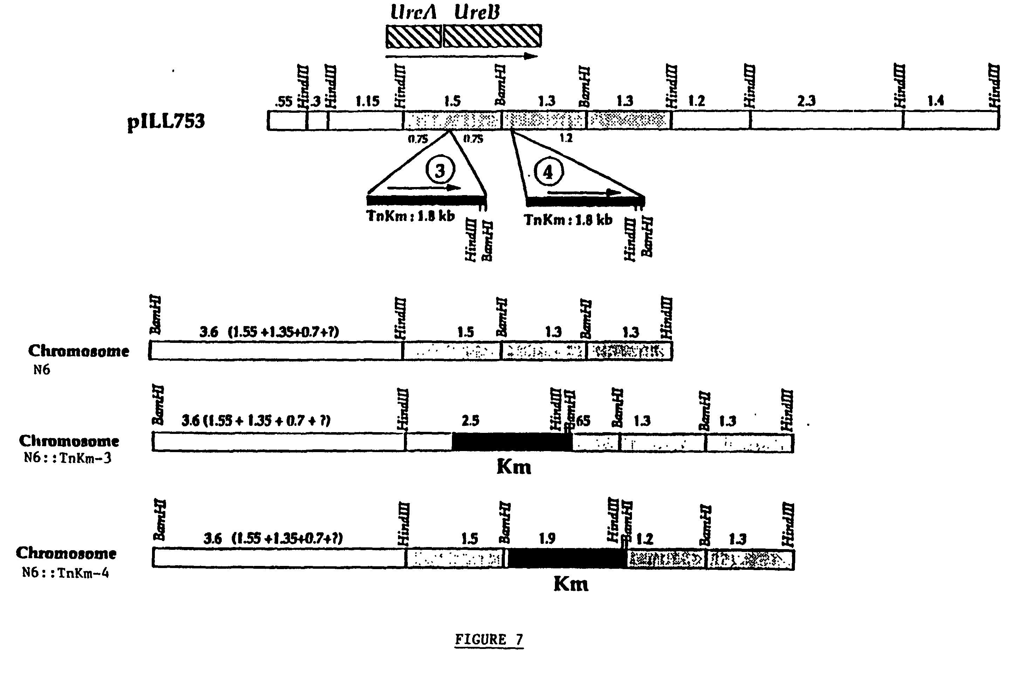

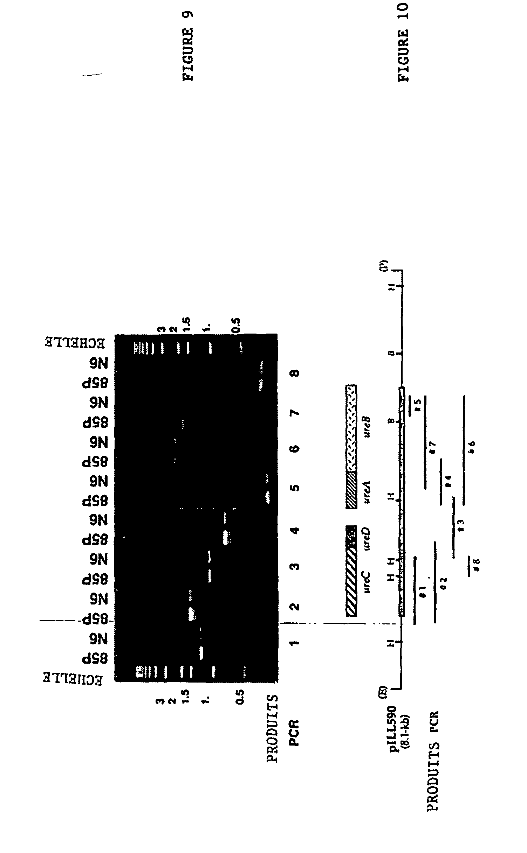

*   l'analyse par hybridation type Southern des profils de restriction par BamHI et HindIII des ADN des souches parentales et mutées témoigne du remplacement des gènes (figure 7 et son interprétation figure 6).
    Une des difficultés rencontrées provient du fait que la souche transformée (N6) n'est pas celle à partir de laquelle le clonage des gènes de l'uréase a été réalisé, cette dernière souche étant la souche 85P et que les sites de restriction HindIII et BamHI ne sont pas conservés d'une souche à l'autre : une sonde correspondant au fragment de 8,1 kb provenant de pILL590 (figure 1) montre clairement des profils de restrictions HindIII qui diffèrent entre N6 et 85P (figure 9), en particulier absence des fragments de 1,25 kb et 1,15 kb. Par contre les fragments HindIII de 4,1 kb et BamHI de 5,1 et de 1,3 kb sont conservés. Il a donc été vérifié par amplification enzymatique (PCR) à l'aide d'oligonucléotides répartis sur l'ensemble de la région correspondant aux gènes ureA, ureB, ureC et ureD) que les produits d'amplification 1 à 6 montrés figure 10 étaient les mêmes dans les deux souches, et que l'absence des sites de restrictions HindIII reflétait le polymorphisme génique et non un réarrangement majeur de la région uréase. Une telle vérification faite, il est possible de confirmer sans ambiguïté le remplacement génique de l'allèle sauvage par l'allèle muté dans les deux mutants créés.

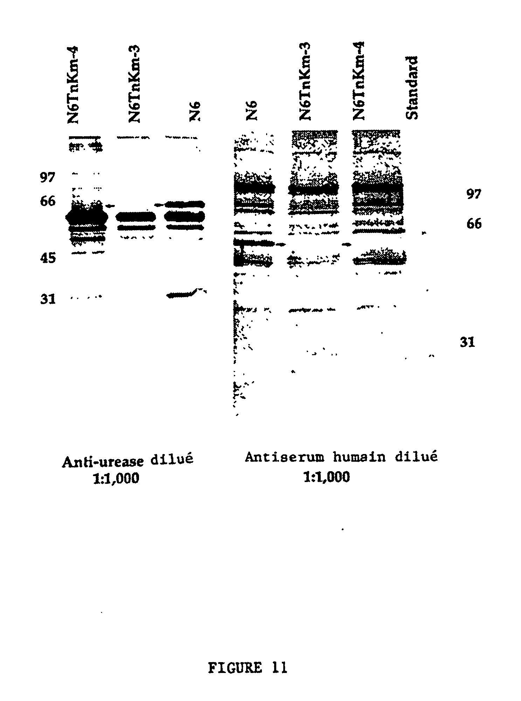

*   enfin il a été vérifié par immunobuvardage à l'aide d'anticorps anti-uréase, ou anti H. pylori préparés chez le lapin, ou anti-H. pylori présents dans le sérum de patients infectés par H. pylori, que les souches mutées N6::TnKm-3 etN6::TnKm-4 n'exprimaient plus le polypeptide de 61 kDaltons codé par le gène ureB et donc que le gène ureB de ces souches avait bien été interrompu (figure 11).

## Revendications

**1.** Séquence nucléotidique, **caractérisée en ce qu'**il s'agit de

(a) une ou plusieurs des séquences nucléotidiques constituées par les gènes appelés ureE, ureF, ureG, ureH et ureI tels que présentés à la figure 4, ou
(b) une séquence d'au moins 15 nucléotides codant pour toute partie de l'un des polypeptides UreE, UreF, UreG, UreH ou UreI reconnue par des anticorps dirigés contre H. pylori ou capable de se comporter comme un haptène ou un immunogène,
(c) une séquence d'au moins 15 nucléotides homologue d'une séquence d'un gène ureE, ureF, ureG, ureH ou ureI tel que défini en (a) et hybridant dans des conditions stringentes avec cette séquence, ladite séquence homologue étant modifiée par délétion, addition, substitution ou inversion d'un ou plusieurs nucléotides de telle façon que le polypeptide codé par cette séquence modifiée conserve une même contribution à la formation d'une uréase fonctionnelle chez H. pylori que celle observée pour le polypeptide correspondant codé par la séquence non modifiée du gène ureE, ureF, ureG, ureH, ou ureI, tel qu'exprimé chez H. pylori,
(d) une séquence complémentaire de l'une des séquences nucléotidiques définies en (a), (b) ou (c).

**2.** Séquence nucléotidique, **caractérisée en ce qu'**elle est constituée par l'ensemble des séquences nucléotidiques des gènes ureE, ureF, ureG, ureH et ureI tels que présentés à la figure 4, ou, l'ensemble formé par des séquences nucléotidiques variantes correspondant à ces gènes modifiés par délétion, addition, substitution ou inversion d'un ou plusieurs nucléotides, indépendamment les uns des autres, de telle façon que l'ensemble de ces variantes code pour des polypeptides conservant une même contribution à la formation d'une uréase fonctionnelle chez H. pylori que celle observée avec l'ensemble des polypeptides correspondants codés par les séquences non modifiées des gènes ureE, ureF, ureG, ureH ou ureI, tels qu'exprimés chez H. pylori, ou une séquence complémentaire de l'une des séquences définies ci-dessus.

**3.** Séquence nucléotidique, **caractérisée en ce qu'**il s'agit d'une séquence d'une taille d'au moins 15 nucléotides hybridant dans des conditions stringentes avec une séquence d'un gène ureE, ureF, ureG ou ureH selon la revendication 1, ou leur séquence complémentaire, ladite séquence étant modifiée par délétion, addition, substitution ou inversion d'un ou plusieurs nucléotides de telle façon que cette séquence ne permet pas l'expression d'une uréase fonctionnelle chez une souche de H. pylori mutée par remplacement allèlique de sa séquence sauvage par ladite séquence nucléotidique correspondante.

**4.** Séquence nucléotidique selon la revendication 1 ou 2, **caractérisée en ce qu'**elle est nécessaire à l'expression de l'uréase fonctionnelle dans des cellules de E. coli préalablement transformées avec les gènes de structure ureA, ureB, ureC et ureD.

**5.** Séquence nucléotidique selon la revendication 3, **caractérisée en ce qu'**elle est associée aux séquences nucléiques correspondant aux gènes de structure ureA et ureB codant pour les sous-unités uréasiques chez H. pylori.

**6.** Séquence nucléotidique selon la revendication 3, **caractérisée en ce qu'**elle est associée aux gènes ureA, ureB, ureC et/ou ureD codant pour l'uréase chez H. pylori.

**7.** Séquence nucléotidique selon l'une des revendications 1 ou 2, **caractérisée en ce qu'**il s'agit de l'enchaînement ureE correspondant aux nucléotides 800 à 1309 de la séquence de la figure 4, ou à tout fragment de cet enchaînement dès lors qu'il hybride dans des conditions stringentes avec l'enchaînement ureE ou avec la séquence complémentaire.

**8.** Séquence nucléotidique selon l'une des revendications 1 ou 2, **caractérisée en ce qu'**il s'agit de l'enchaînement ureF correspondant aux nucléotides 1324 à 2091 de la séquence de la figure 4, ou à tout fragment de cet enchaînement dès lors qu'il hybride dans des conditions stringentes avec l'enchaînement ureF ou avec la séquence complémentaire.

**9.** Séquence nucléotidique selon l'une des revendications 1 ou 2, **caractérisée en ce qu'**il s'agit de l'enchaînement ureG correspondant aux nucléotides 2123 à 2719 de la séquence de la figure 4, ou à tout fragment de cet enchaînement dès lors qu'il hybride dans des conditions stringentes avec l'enchaînement ureG ou avec la séquence complémentaire.

**10.** Séquence nucléotidique selon l'une des revendications 1 ou 2, **caractérisée en ce qu'**il s'agit de l'enchaînement ureH correspondant aux nucléotides 2722 à 3516 de la séquence de la figure 4, ou à tout fragment de cet enchaînement dès lors qu'il hybride dans des conditions stringentes avec l'enchaînement ureH ou avec la séquence complémentaire.

**11.** Séquence nucléotidique selon l'une des revendications 1 ou 2, **caractérisée en ce qu'**il s'agit de l'enchaînement ureI correspondant aux nucléotides 211 à 795 de la séquence de la figure 4, ou à tout fragment de cet enchaînement dès lors qu'il hybride dans des conditions stringentes avec l'enchaînement ureI ou avec la séquence complémentaire.

**12.** Séquence nucléotidique selon la revendication 7. **caractérisée en ce qu'**il s'agit de l'enchaînement nucléotidique suivant ou **en ce qu'**il comprend cet enchaînement :

GCG AAA ATA TGC TAT GAA ATA GGA AAC CGC CAT

**13.** Sonde nucléotidique **caractérisée en ce qu'**elle est constituée par une séquence nucléotidique selon l'une quelconque des revendications 1 à 12, ladite séquence étant marquée.

14. Amorce nucléotidique, **caractérisée en ce qu'**elle comprend un fragment nucléotidique d'une séquence selon l'une quelconque des revendications 1 à 12, ledit fragment comprenant environ, 18 à environ 30, de préférence environ 25 à environ 30 nucléotides, et étant capable d'hybrider aux extrémités 5' et 3' d'un fragment nucléotidique spécifique d'au moins un gène choisi parmi ureE, ureF, ureG ou ureH, pour l'utilisation dans une réaction d'amplification génique.

15. Utilisation d'une amorce selon la revendication 14, pour la détedion *in vitro* d'une infection par H. pylori, à partir d'un échantillon biologique et à l'issue de réactions d'amplification génique.

16. Utilisation d'une sonde selon la revendication 13, pour la détection *in vitro* dans un échantillon biologique, d'une infection par H. pylori, le cas échéant à l'issue de réactions d'amplification génique.

17. Polypeptide, **caractérisé en ce qu'**il s'agit

    i) de l'un des polypeptides UreE, UreF, UreG, UreH ou UreI présenté à la figure 4, ou,
    ii) toute partie d'au moins un des polypeptides définis en (i) et conservant une même contribution à la formation d'une uréase fonctionnelle chez H. pylori que celle observée pour les polypeptides correspondants non modifiés UreE, UreF; UreG, UreH et UreI tels qu'exprimés chez H. pylori,
    iii) toute partie d'au moins un des polypeptides définis en (i) reconnue par des anticorps dirigés contre H. pylori ou capable de se comporter comme un haptène ou un immunogène.

18. Polypeptide selon la revendication 17, modifié par délétion, addition, substitution ou inversion d'un ou plusieurs acides aminés, dès lors qu'il conserve une même contribution à la formation d'un uréase fonctionnelle chez H. pylori que celle observée pour les polypeptides UreE, UreF, UreG, UreH, et UreI tels qu'exprimés chez H. pylori.

19. Vecteur recombinant, **caractérisé en ce qu'**il contient une séquence selon la revendication 3.

20. Vecteur recombinant selon la revendication 19, **caractérisé en ce qu'**il s'agit d'un cosmide ou d'un plasmide.

21. Vecteur recombinant selon la revendication 19 ou 20, **caractérisé en ce qu'**il s'agit du plasmide pILL753 contenu dans E. coli HB101, déposé à la CNCM le 3 octobre 1991 sous le numéro I-1148.

22. Vecteur recombinant selon la revendication 19 ou 20, **caractérisé en ce qu'**il s'agit du plasmide pILL763 contenu dans E. coli HB101 déposé à la CNCM le 3 octobre 1991 sous le numéro I-1149.

23. Souche de H. pylori recombinante, **caractérisée en ce qu'**elle présente une mutation au niveau d'au moins un gène choisi parmi les gènes ureE, ureF, ureG, ureH ou ureI présenté à la figure 4, dans des conditions telles que la souche recombinante exprime un phénotype uréase-négatif, ou présente une atténuation de l'activité uréasique par rapport à la même souche non mutée.

24. Souche de H. pylori recombinante selon la revendication 23, **caractérisée en ce qu'**elle est mutée au niveau du gène ureG.

25. Souche de H. pylori recombinante selon la revendication 23 ou 24. **caractérisée en ce qu'**il s'agit de la souche N6 (NCIMB n°40512) mutée.

26. Souche de H. pylori recombinante selon l'une des revendications 23 à 25, **caractérisée en ce qu'**elle comprend une séquence nucléotidique selon la revendication 3.

27. Souche de H. pylori recombinante selon la revendication 25, **caractérisée en qu'**il s'agit d'une souche N6 mutée au niveau de l'un au moins des gènes ureE, ureF, ureG, ureH ou ureI par remplacement allèlique de sa séquence sauvage par la séquence modifiée correspondante selon la revendication 3. de telle sorte que son activité uréasique est atténuée par rapport à la même souche non mutée.

28. Hôte cellulaire recombinant, différent de H. pylori, **caractérisé en ce qu'**il est transformé par une séquence selon l'une quelconque des revendications 1 à 12 ou un vecteur recombinant selon l'une des revendications 19 à 22, dans des conditions permettant son expression dans l'hôte.

**29.** Hôte cellulaire recombinant selon la revendication 28, **caractérisé en ce qu'**il s'agit d'une souche E. coli recombinante.

**30.** Composition immunogène, **caractérisée en ce qu'**elle contient un hôte cellulaire recombinant selon l'une quelconque des revendications 28 à 29 ou une souche de H. pylori recombinante selon l'une quelconque des revendications 23 à 27.

**31.** Kit pour le diagnostic in vitro d'une infection par H. pylori sur un échantillon biologique déterminé, **caractérisé en ce qu'**il comprend :

- au moins un couple d'amorces nucléotidiques selon la revendication capables d'hybrider aux extrémités 5' et 3' d'un fragment nucléotidique spécifique d'au moins un gène choisi parmi ureE, ureF, ureG, ureH, ou ureI,
- des réactifs nécessaires à l'extraction des acides nucléiques à partir de l'échantillon traité,
- des réactifs pour effectuer la polymérisation dudit fragment nucléotidique, à partir des amorces nucléotidiques, notamment des enzymes de polymérisation, en quantité suffisante pour réaliser l'amplification du fragment que l'on souhaite amplifier,
- au moins un enchaînement de nucléotides pouvant être utilisé comme sonde et capable d'hybrider dans des conditions déterminées avec le fragment nucléotidique amplifié,
- le cas échéant des moyens pour révéler l'hybridation.

**32.** Kit pour le diagnostic in vitro d'une infection par H. pylori, **caractérisé en ce qu'**il comprend :

- une quantité déterminée de sonde selon la revendication 13,
- un milieu approprié pour la réalisation d'une réaction d'hybridation entre l'acide nucléique de H. pylori à détecter et la sonde,
- des réactifs pour la détection des hybrides éventuellement formés.

**33.** Anticorps polyclonal ou monoclonal, **caractérisé en ce qu'**il reconnaît un polypeptide selon l'une quelconque des revendications 17 ou 18.

**34.** Composition pour le traitement d'une infection par H. pylori, **caractérisée en ce qu'**elle contient un anticorps selon la revendication 33.

**35.** Polypeptide constitué par l'enchaînement : Ala Lys Ile Cys Tyr Glu Ile Gly Asn Arg His.

**Patentansprüche**

**1.** Nukleotidsequenz, **dadurch gekennzeichnet, dass** es sich um

(a) eine oder mehrere der Nukleotidsequenzen, die aus den ureE, ureF, ureG, ureH und ureI genannten, wie in der Figur 4 gezeigten Genen bestehen, oder

(b) eine Sequenz von wenigstens 15 Nukleotiden, die für jeden Teil eines der Polypeptide UreE, UreF, UreG, UreH oder UreI kodiert, das von gegen H. pylori gerichteten Antikörpern erkannt wird oder fähig ist, sich wie ein Hapten oder ein Immunogen zu verhalten,

(c) eine Sequenz von wenigstens 15 Nukleotiden, die homolog zu einer Sequenz eines wie in (a) definierten Gens ureE, ureF, ureG, ureH oder ureI ist und unter stringenten Bedingungen mit dieser Sequenz hybridisiert, wobei die homologe Sequenz durch Deletion, Addition, Substitution oder Inversion eines oder mehrerer Nukleotide in der Weise modifiziert ist, dass das von dieser modifizierten Sequenz kodierte Polypeptid einen gleichen Beitrag zu der Bildung einer funktionellen Urease bei H. pylori bewahrt wie der, der für das entsprechende Polypeptid beobachtet wird, das von der nicht-modifizierten Sequenz des Gens ureE, ureF, ureG, ureH oder ureI kodiert wird, wie es bei H. pylori exprimiert wird,

(d) eine komplementäre Sequenz einer der in (a), (b) oder (c) definierten Nukleotidsequenzen

handelt.

**2.** Nukleotidsequenz, **dadurch gekennzeichnet, dass** sie aus der Gruppe der Nukleotidsequenzen der wie in der Figur 4 gezeigten Gene ureE, ureF, ureG, ureH und ureI besteht oder der von Nukleotidsequenz-Varianten gebildeten Gruppe, die diesen Genen entspricht, die durch Deletion, Addition, Substitution oder Inversion eines oder mehrerer Nukleotide unabhängig voneinander in der Weise modifiziert sind, dass die Gruppe dieser Varianten für Polypeptide kodiert, die einen gleichen Beitrag zu der Bildung einer funktionellen Urease bei H. pylori bewahren wie der, der mit der Gruppe der entsprechenden, von den nicht-modifizierten Sequenzen der Gene ureE, ureF, ureG, ureH oder ureI kodierten Polypeptide beobachtet wird, wie sie bei H. pylori exprimiert werden, oder einer komplementären Sequenz einer der oben definierten Sequenzen.

**3.** Nukleotidsequenz, **dadurch gekennzeichnet, dass** es sich um eine Sequenz mit einer Länge von wenigstens 15 Nukleotiden handelt, die unter stringenten Bedingungen mit einer Sequenz eines Gens ureE, ureF, ureG oder ureH nach Anspruch 1 hybridisiert, oder deren komplementäre Sequenz, wobei die Sequenz durch Deletion, Addition, Substitution oder Inversion eines oder mehrerer Nukleotide in der Weise modifiziert ist, dass diese Sequenz die Expression einer funktionellen Urease bei einem Stamm von H. pylori nicht erlaubt, der durch allelischen Austausch seiner Wildtyp-Sequenz durch die genannte entsprechende Nukleotidsequenz mutiert ist.

**4.** Nukleotidsequenz nach Anspruch 1 oder 2, **dadurch gekennzeichnet, dass** sie für die Expression der funktionellen Urease in Zellen von E. coli erforderlich ist, die zuvor mit den Strukturgenen ureA, ureB, ureC und ureD transformiert wurden.

**5.** Nukleotidsequenz nach Anspruch 3, **dadurch gekennzeichnet, dass** sie mit den Nukleinsequenzen assoziiert ist, die den Strukturgenen ureA und ureB entsprechen, die für die Urease-Untereinheiten bei H. pylori kodieren.

**6.** Nukleotidsequenz nach Anspruch 3, **dadurch gekennzeichnet, dass** sie mit den für die Urease bei H. pylori kodierenden Genen ureA, ureB, ureC und/oder ureD assoziiert ist.

**7.** Nukleotidsequenz nach einem der Ansprüche 1 oder 2, **dadurch gekennzeichnet, dass** es sich um die Kette ureE handelt, die den Nukleotiden 800 bis 1309 der Sequenz der Figur 4 entspricht, oder um jedes Fragment dieser Kette, sofern es unter stringenten Bedingungen mit der Kette ureE oder mit der komplementären Sequenz hybridisiert.

**8.** Nukleotidsequenz nach einem der Ansprüche 1 oder 2, **dadurch gekennzeichnet, dass** es sich um die Kette ureF handelt, die den Nukleotiden 1324 bis 2091 der Sequenz der Figur 4 entspricht, oder um jedes Fragent dieser Kette, sofern es unter stringenten Bedingungen mit der Kette ureF oder mit der komplementären Sequenz hybridisiert.

**9.** Nukleotidsequenz nach einem der Ansprüche 1 oder 2, **dadurch gekennzeichnet, dass** es sich um die Kette ureG handelt, die den Nukleotiden 2123 bis 2719 der Sequenz der Figur 4 entspricht, oder um jedes Fragment dieser Kette, sofern es unter stringenten Bedingungen mit der Kette ureG oder mit der komplementären Sequenz hybridisiert.

**10.** Nukleotidsequenz nach einem der Ansprüche 1 bis 2, **dadurch gekennzeichnet, dass** es sich um die Kette ureH handelt, die den Nukleotiden 2722 bis 3516 der Sequenz der Figur 4 entspricht, oder um jedes Fragment dieser Kette, sofern es unter stringenten Bedingungen mit der Kette ureH oder mit der komplementären Sequenz hybridisiert.

**11.** Nukleotidsequenz nach einem der Ansprüche 1 oder 2, **dadurch gekennzeichnet, dass** es sich um die Kette ureI handelt, die den Nukleotiden 211 bis 795 der Sequenz der Figur 4 entspricht, oder um jedes Fragment dieser Kette, sofern es unter stringenten Bedingungen mit der Kette ureI oder mit der komplementären Sequenz hybridisiert.

**12.** Nukleotidsequenz nach Anspruch 7, **dadurch gekennzeichnet, dass** es sich um die folgende Nukleotidkette handelt oder dass sie diese Kette umfasst:

GCG AAA ATA TGC TAT GAA ATA GGA AAC CGC CAT.

**13.** Nukleotidsonde, **dadurch gekennzeichnet, dass** sie aus einer Nukleotidsequenz nach einem beliebigen der Ansprüche 1 bis 12 besteht, wobei die Sequenz markiert ist.

14. Nukleotidstarter, **dadurch gekennzeichnet, dass** er ein Nukleotidfragment mit einer Sequenz nach einem beliebigen der Ansprüche 1 bis 12 umfasst, wobei das Fragment in etwa 18 bis in etwa 30, vorzugsweise in etwa 25 bis in etwa 30 Nukleotide umfasst und fähig ist, an die 5'- und 3'-Enden eines Nukleotidfragments zu hybridisieren, das spezifisch für wenigstens ein Gen, ausgewählt aus ureE, ureF, ureG, oder ureH ist, zur Verwendung in einer Genamplifikations-Reaktion.

15. Verwendung eines Starters nach Anspruch 14 für den In-vitro-Nachweis einer Infektion durch H. pylori, ausgehend von einer biologischen Probe und nach Genamplifikations-Reaktionen.

16. Verwendung einer Sonde nach Anspruch 13 für den In-vitro-Nachweis einer Infektion durch H. pylori in einer biologischen Probe, gegebenenfalls nach Genamplifikations-Reaktionen.

17. Polypeptid, **dadurch gekennzeichnet, dass** es sich um

   i) eines der in Figur 4 gezeigten Polypeptide UreE, UreF, UreG, UreH oder UreI, oder
   ii) jeden Teil wenigstens eines der in (i) definierten Polypeptide, der einen eigenen Beitrag zu der Bildung einer funktionellen Urease bei H. pylori bewahrt wie der, der bei den entsprechenden nicht-modifizierten, wie bei H. pylori exprimierten Polypeptiden UreE, UreF, UreG, UreH und UreI beobachtet wird,
   iii) jeden Teil wenigstens eines der in (i) definierten Polypeptide, der von gegen H. pylori gerichteten Antikörpern erkannt wird oder fähig ist, sich wie ein Hapten oder ein Immunogen zu verhalten,

   handelt.

18. Polypeptid nach Anspruch 17, das durch Deletion, Addition, Substitution oder Inversion einer oder mehrerer Aminosäuren modifiziert ist, sofern es einen eigenen Beitrag zu der Bildung einer funktionellen Urease bei H. pylori bewahrt wie der, der bei den Polypeptiden UreE, UreF, UreG, UreH und UreI beobachtet wird, wie sie bei H. pylori exprimiert werden.

19. Rekombinanter Vektor, **dadurch gekennzeichnet, dass** er eine Sequenz nach Anspruch 3 enthält.

20. Rekombinanter Vektor nach Anspruch 19, **dadurch gekennzeichnet, dass** es sich um ein Cosmid oder um ein Plasmid handelt.

21. Rekombinanter Vektor nach Anspruch 19 oder 20, **dadurch gekennzeichnet, dass** es sich um das in E. coli HB101 enthaltene, bei der CNCM am 3. Oktober 1991 unter der Nummer I-1148 hinterlegte Plasmid pILL753 handelt.

22. Rekombinanter Vektor nach Anspruch 19 oder 20, **dadurch gekennzeichnet, dass** es sich um das in E. coli HB101 enthaltene, bei der CNCM am 3. Oktober 1991 unter der Nummer I-1149 hinterlegte Plasmid pILL763 handelt.

23. Rekombinanter Stamm von H. pylori, **dadurch gekennzeichnet, dass** er eine Mutation auf der Stufe wenigstens eines Gens aufweist, das aus den in der Figur 4 gezeigten Genen ureE, ureF, ureG, ureH oder ureI ausgewählt ist, unter solchen Bedingungen, dass der rekombinante Stamm einen Urease-negativen Phenotyp exprimiert oder eine Verringerung der Ureaseaktivität im Vergleich zu dem gleichen, nicht-mutierten Stamm aufweist.

24. Rekombinanter Stamm von H. pylori nach Anspruch 23, **dadurch gekennzeichnet, dass** er auf der Stufe des Gens ureG mutiert ist.

25. Rekombinanter Stamm von H. pylori nach Anspruch 23 oder 24, **dadurch gekennzeichnet, dass** es sich um den mutierten Stamm N6 (NCIMB Nr. 40512) handelt.

26. Rekombinanter Stamm von H. pylori nach einem der Ansprüche 23 bis 25, **dadurch gekennzeichnet, dass** er eine Nukleotidsequenz nach Anspruch 3 umfasst.

27. Rekombinanter Stamm von H. pylori nach Anspruch 25, **dadurch gekennzeichnet, dass** es sich um einen Stamm N6 handelt, der auf der Stufe wenigstens eines der Gene ureE, ureF, ureG, ureH oder ureI durch allelischen Austausch seiner Wildtyp-Sequenz durch die modifizierte entsprechende Sequenz nach Anspruch 3 in der Weise mutiert ist, dass seine Ureaseaktivität im Vergleich zu dem gleichen nicht-mutierten Stamm abgeschwächt ist.

28. Rekombinanter zellulärer Wirt, verschieden von H. pylori, **dadurch gekennzeichnet, dass** er mit einer Sequenz nach einem beliebigen der Ansprüche 1 bis 12 oder einem rekombinanten Vektor nach einem der Ansprüche 19 bis 22 unter Bedingungen, die seine Expression in dem Wirt erlauben, transformiert ist.

29. Rekombinanter zellulärer Wirt nach Anspruch 28, **dadurch gekennzeichnet, dass** es sich um einen rekombinanten Stamm von E. coli handelt.

30. Immunogene Zusammensetzung, **dadurch gekennzeichnet, dass** sie einen rekombinanten zellulären Wirt nach einem beliebigen der Ansprüche 28 bis 29 oder einen rekombinanten Stamm von H. pylori nach einem beliebigen der Ansprüche 23 bis 27 enthält.

31. Kit für die In-vitro-Diagnose einer Infektion durch H. pylori in einer bestimmten biologischen Probe, **dadurch gekennzeichnet, dass** es umfasst:

   - wenigstens ein Paar Nukleotidstarter nach Anspruch 14, die fähig sind, an die 5'- und 3'-Enden eines spezifischen Nukleotidfragments wenigstens eines Gens ausgewählt aus ureE, ureF, ureG, ureH oder ureI zu hybridisieren,
   - erforderliche Reagenzien für die Extraktion von Nukleinsäuren, ausgehend von der behandelten Probe,
   - Reagenzien für die Durchführung der Polymerisation des Nukleotidfragments, ausgehend von den Nukleotidstartern, inbesondere Polymerisierungsenzyme, in ausreichender Menge, um die Amplifizierung des Fragments durchzuführen, das man zu amplifizieren wünscht,
   - wenigstens eine Nukleotidkette, die als Sonde verwendet werden kann und fähig ist, unter bestimmten Bedingungen mit dem amplifizierten Nukleotidfragment zu hybridisieren,
   - gegebenenfalls Mittel, um die Hybridisierung festzustellen.

32. Kit für die In-vitro-Diagnose einer Infektion durch H. pylori, **dadurch gekennzeichnet, dass** es umfasst:

   - eine bestimmte Menge Sonde nach Anspruch 13,
   - ein geeignetes Medium für die Durchführung einer Hybridisierungsreaktion zwischen der nachzuweisenden Nukleinsäure von H. pylori und der Sonde,
   - Reagenzien für den Nachweis von eventuell gebildeten Hybriden.

33. Polyklonaler oder monoklonaler Antikörper, **dadurch gekennzeichnet, dass** er ein Polypeptid nach einem beliebigen der Ansprüche 17 oder 18 erkennt.

34. Zusammensetzung für die Behandlung einer Infektion durch H. pylori, **dadurch gekennzeichnet, dass** sie einen Antikörper nach Anspruch 33 enthält.

35. Polypeptid, bestehend aus der Kette: Ala Lys Ile Cys Tyr Glu Ile Gly Asn Arg His.

**Claims**

1. A nucleotide sequence, **characterized in that** it is:

   a) one or more nucleotide sequences constituted by genes termed ureE, ureF, ureG, ureH and ureI as shown in Figure 4; or
   b) a sequence of at least 15 nucleotides encoding all or a portion of one of the polypeptides UreE, UreF, UreG, UreH or UreI recognized by antibodies directed against H. pylori or capable of behaving as a hapten or immunogen;
   c) a sequence of at least 15 nucleotides homologous with a sequence of a ureE, ureF, ureG, ureH or ureI gene as defined in a) and hybridizing under stringent conditions with said sequence, said homologous sequence being modified by deletion, addition, substitution or inversion of one or more nucleotides such that the polypeptide encoded by said modified sequence conserves the same contribution to the formation of a functional urease in H. pylori as that observed for the corresponding polypeptide encoded by the unmodified sequence of the ureE, ureF, ureG, ureH or ureI gene as expressed in H/ pylori;
d) a complementary sequence of one of the nucleotide sequences defined in a), b) or c).

2. A nucleotide sequence, **characterized in that** it is constituted by the ensemble of nucleotide sequences for the genes ureE, ureF, ureG, ureH and ureI as shown in Figure 4 or the ensemble formed by variant nucleotide sequences corresponding to said genes modified by deletion, addition, substitution or inversion of one or more nucleotides, independently of each other, such that the ensemble of said variants encodes polypeptides conserving the same contribution to the formation of a functional urease in H. pylori as that observed with the ensemble of corresponding polypeptides encoded by the unmodified sequences for the ureE, ureF, ureG, ureH or ureI genes as expressed in H. pylori, or a complementary sequence of one of the sequences defined above.

3. A nucleotide sequence, **characterized in that** it is a sequence with a size of at least 15 nucleotides hybridizing under stringent conditions with a sequence of a ureE, ureF, ureG or ureH gene in accordance with claim 1 or a complementary sequence thereof, said sequence being modified by deletion, addition, substitution or inversion of one or more nucleotides such that said sequence does not allow expression of a functional urease in a strain of H. pylori mutated by allelic replacement of the wild type sequence by said corresponding nucleotide sequence.

4. A nucleotide sequence according to claim 1 or claim 2, **characterized in that** it is necessary for expression of functional urease in cells of E. coli previously transformed with genes with structure ureA, ureB, ureC and ureD.

5. A nucleotide sequence according to claim 3, **characterized in that** it is associated with nucleic sequences corresponding to genes with structure ureA and ureB encoding urease sub-units in H. pylori.

6. A nucleotide sequence according to claim 3, **characterized in that** it is associated with genes ureA, ureB, ureC and/or ureD encoding for urease in H. pylori.

7. A nucleotide sequence according to claim 1 or claim 2, **characterized in that** it is the ureE concatenation corresponding to nucleotides 800 to 1309 of the sequence of Figure 4, or to any fragment of said concatenation provided that it hybridizes under stringent conditions with the ureE concatenation or with the complementary sequence.

8. A nucleotide sequence according to claim 1 or claim 2, **characterized in that** it is the ureF sequence corresponding to nucleotides 1324 to 2091 of the sequence of Figure 4, or to any fragment of said concatenation provided that it hybridizes under stringent conditions with the ureF concatenation or with the complementary sequence.

9. A nucleotide sequence according to claim 1 or claim 2, **characterized in that** it is the ureG concatenation corresponding to nucleotides 2123 to 2719 of the sequence of Figure 4 or to any fragment of said concatenation provided that it hybridizes under stringent conditions with the ureG concatenation or with the complementary sequence.

10. A nucleotide sequence according to claim 1 or claim 2, **characterized in that** it is the ureH concatenation corresponding to nucleotides 2722 to 3516 of the sequence of Figure 4, or to any fragment of said concatenation provided that it hybridizes under stringent conditions with the ureH concatenation or with the complementary sequence.

11. A nucleotide sequence according to claim 1 or claim 2, **characterized in that** it is the ureI concatenation corresponding to nucleotides 211 to 795 of the sequence of Figure 4, or to any fragment of said concatenation provided that it hybridizes under stringent conditions with the ureI concatenation or with the complementary sequence.

12. A nucleotide sequence according to claim 7, **characterized in that** it is the following nucleotide concatenation or **in that** it comprises said concatenation:

GCG AAA ATA TGC TAT GAA ATA GGA AAC CGC CAT.

13. A nucleotide probe, **characterized in that** it is constituted by a nucleotide sequence according to any one of claims 1 to 12, said sequence being labeled.

14. A nucleotide primer, **characterized in that** it comprises a nucleotide fragment of a sequence according to any one of claims 1 to 12, said fragment comprising about 18 to about 30, preferably about 25 to about 30 nucleotides, and is capable of hybridizing to the 5' and 3' ends of a specific nucleotide fragment of at least one gene selected from ureE, ureF, ureG or ureH for use in a gene amplification reaction.

15. Use of a primer according to claim 14, for *in vitro* detection of an infection with H. pylori, in a biological sample and following gene amplification reactions.

16. Use of a probe according to claim 13, for in vitro detection in a biological sample of an infection with H. pylori, if necessary following gene amplification reactions.

17. A polypeptide, **characterized in that** it is:

i) one of the polypeptides UreE, UreF, UreG, UreH or UreI shown in Figure 4; or
ii) all parts of at least one of the polypeptides defined in i) and conserving the same contribution to the formation of a functional urease in H. pylori as that observed for the corresponding unmodified polypeptides UreE, UreF, UreG, UreH and UreI as expressed in H. pylori;
iii) all parts of at least one of the polypeptides defined in i) recognized by antibodies directed against H. pylori or capable of behaving as a hapten or immunogen.

18. A polypeptide according to claim 17, modified by deletion, addition, substitution or inversion of one or more amino acids provided that it conserves the same contribution to the formation of a functional urease in H. pylori as that observed for the polypeptides UreE, UreF, UreG, UreH and UreI as expressed in H. pylori.

19. A recombinant vector, **characterized in that** it contains a sequence according to claim 3.

20. A recombinant vector according to claim 19, **characterized in that** it is a cosmid or a plasmid.

21. A recombinant vector according to claim 19 or claim 20, **characterized in that** it is the plasmid pILL753 contained in E. coli HB101 deposited at the CNCM on 3rd October 1991 with accession number I-1148.

22. A recombinant vector according to claim 19 or claim 20, **characterized in that** it is the plasmid pILL763 contained in E. coli HB101 deposited at the CNCM on 3rd October 1991 with accession number I-1149.

23. A recombinant strain of H. pylori, **characterized in that** it has a mutation in at least one gene selected from genes ureE, ureF, ureG, ureH or ureI shown in Figure 4, under conditions such that the recombinant strain expresses a urease-negative phenotype or exhibits attenuation of urease activity compared with the same non-mutated strain.

24. A recombinant strain of H. pylori according to claim 23, **characterized in that** it is mutated at the ureG gene.

25. A recombinant strain of H. pylori according to claim 23 or claim 24, **characterized in that** it is the mutated N6 strain (NCIMB n° 40512).

26. A recombinant strain of H. pylori according to one of claims 23 to 25, **characterized in that** it comprises a nucleotide sequence according to claim 3.

27. A recombinant strain of H. pylori according to claim 25, **characterized in that** it is a N6 strain mutated in at least one of the ureE, ureF, ureG, ureH or ureI genes by allelic replacement of its wild type sequence by the corresponding modified sequence according to claim 3, such that its urease activity is attenuated with respect to the same non mutated strain.

28. A recombinant host cell different from H. pylori, **characterized in that** it is transformed by a sequence according to any one of claims 1 to 12 or a recombinant vector according to one of claims 19 to 22, under conditions allowing its expression in the host.

29. A recombinant host cell according to claim 28, **characterized in that** it is a recombinant E. coli strain.

30. An immunogenic composition, **characterized in that** it contains a recombinant host cell according to claim 28 or claim 29 or a recombinant strain of H. pylori according to any one of claims 23 to 27.

31. A kit for *in vitro* diagnosis of any infection with H. pylori in a predetermined biological sample, **characterized in that** it comprises:

   - at least one pair of nucleotide primers according to claim 14 that are capable of hybridizing to the 5' and 3' ends of a specific nucleotide fragment of at least one gene selected from ureE, ureF, ureG, ureH and ureI;
   - reagents necessary for extracting nucleic acids from the treated sample;
   - reagents to carry out polymerization of said nucleotide fragment from nucleotide primers, in particular polymerization enzymes in sufficient quantity to carry out amplification of the fragment which is to be amplified;
   - at least one concatenation of nucleotides which can be used as a probe and which is capable of hybridizing under predetermined conditions with the amplified nucleotide fragment;
   - if appropriate, means for revealing hybridization.

32. A kit for *in vitro* diagnosis of an infection with H. pylori, **characterized in that** it comprises:

   - a predetermined quantity of a probe according to claim 13;
   - a suitable medium for carrying out a hybridization reaction between the nucleic acid of H. pylori to be detected and the probe;
   - reagents for detecting any hybrids which are formed.

33. A polyclonal or monoclonal antibody, **characterized in that** it recognizes a polypeptide according to claim 17 or claim 18.

34. A composition for treating an infection with H. pylori, **characterized in that** it contains an antibody according to claim 33.

35. A polypeptide constituted by the concatenation: Ala Lys Ile Cys Tyr Glu Ile Gly Asn Arg His.

FIGURE 1

región de *E.coli* contenant l'ADN de l'uréase

EP 0 610 322 B1

FIGURE 2

EP 0 610 322 B1

(A)

B PvuII      B      SpH1      H      H

.1 kb    1.2 kb      .48 kb      .82 kb      1.2 kb

5' ▶ 3'

Amorces

ATG     ATG     ATG     ATG     ATG

(B)

| ureB | ureI | ureE | ureF | ureG | ureH |

O.R.F.

1710 bp    585 bp    510 bp    768 bp    597 bp    795 bp

(C)   UreI: - 195 a.a.    UreE: - 170 a.a.    UreF: - 256 a.a.    UreG: - 199 a.a.    UreH: - 265 a.a.

        - 21 681 D      - 19 461 D      - 28 617 D      - 21 744 D      - 29 650 D

FIGURE 3

FIGURE 4

A CTC TTT AGC ATT TTC TAG GA TTT TTT AGG AGC AAC GCT CTT AGA TCC TTA GTT TTT AGC
--leu phe ser ile phe AMB

TCT CTG ATT TTT TGT TTA TCA AAA AAT TGG GGG CTT TTT TTG TTT TTA TTT TTT GTC AAT

TTA CTA TTT TTC TTT ATG ATT AGC TCA AGC AAC AAA AGT TAT TCG TAA GGT GCG TTT GTT

GTA AAA ATT TTT GTT TGG AAG GAA AAG GCA ATG CTA GGA CTT GTA TTG TTA TAT GTT GGG
       ureI                                   Met leu gly leu val leu leu tyr val gly

ATT GTT TTA ATC AGC AAT GGG ATT TGC GGG TTA ACC AAA GTC GAT CCT AAA AGC ACT GCG
ile val leu ile ser asn gly ile cys gly leu thr lys val asp pro lys ser thr ala

GTG ATG AAC TTT TTT GTG GGT GGG CTC TCC ATT ATT TGT AAT GTG GTT GTC ATC ACT TAT
val met asn phe phe val gly gly leu ser ile ile cys asn val val val ile thr tyr

TCC GCT CTC AAC CCT ACA GCC CCT GTA GAA GGT GCT GAA GAT ATT GCT CAA GTA TCA CAC
ser ala leu asn pro thr ala pro val glu gly ala glu asp ile ala gln val ser his

CAT TTG ACT AAT TTC TAT GGG CCA GCG ACT GGG TTA TTG TTT GGT TTC ACC TAC TTG TAT
his leu thr asn phe tyr gly pro ala thr gly leu leu phe gly phe thr tyr leu tyr

EP 0 610 322 B1

FIGURE 4 (Suite)

481                                                 511
GCG GCT ATC AAC CAC ACT TTT GGT TTG GAT TGG AGG CCC TAC TCT TGG TAT AGC TTA TTC
ala ala ile asn his thr phe gly leu asp trp arg pro tyr ser trp tyr ser leu phe

541                                                 571
GTA GCG ATC AAC ACG ATT CCT GCT GCG ATT TTA TCC CAC TAT AGC GAT ATG CTT GAT GAC
val ala ile asn thr ile pro ala ala ile leu ser his tyr ser asp met leu asp asp

601                                                 631
CAC AAA GTG TTA GGC ATC ACT GAA GGC GAT TGG TGG GCG ATC ATT TGG TTG GCT TGG GGT
his lys val leu gly ile thr glu gly asp trp trp ala ile ile trp leu ala trp gly

661                                                 691
GTT TTG TGG CTT ACC GCT TTC ATT GAA AAC ATC TTG AAA ATC CCT TTA GGG AAA TTC ACT
val leu trp leu thr ala phe ile glu asn ile leu lys ile pro leu gly lys phe thr

721                                                 751
CCA TGG CTT GCT ATC ATT GAG GGC ATT TTA ACC GCT TGG ATC CCT GCT TGG TTA CTC TTT
pro trp leu ala ile ile glu gly ile leu thr ala trp ile pro ala trp leu leu phe

781                                                 811
ATC CAA CAC TGG GTG TGA GAT GAT CAT
ile gln his trp val OPA

782                                                 812
TCC AAC ACT GGG TGT GAG ATG ATC ATA GAG CGT TTA ATA GGC AAT CTA AGG GAT TTA AAC
       ureB          Met ile ile glu arg leu ile gly asn leu arg asp leu asn

FIGURE 4 (suite)

```
842                                                 871
CCC TTG GAT TTC AGC GTG GAT TAT GTG GAT TTG GAA TGG TTT GAA ACG AGG AAA AAA ATC
pro leu asp phe ser val asp tyr val asp leu glu trp phe glu thr arg lys lys ile


902                                                 932
GCT CGC TTT AAA ACC AGG CAA GGC AAA GAC ATA GCC GTA CGC CTT AAA GAC GCT CCC AAG
ala arg phe lys thr arg gln gly lys asp ile ala val arg leu lys asp ala pro lys


962                                                 992
TTG GGT TTC TCT CAA GGA GAT ATT TTA TTT AAA GAA GAG AAG GAA ATT ATC GCC GTT AAT
leu gly phe ser gln gly asp ile leu phe lys glu glu lys glu ile ile ala val asn


1022                                                1052
ATC TTG GAT TCT GAA GTC ATT CAC ATC CAA GCT AAG AGC GTG GCA GAA GTA GCG AAA ATA
ile leu asp ser glu val ile his ile gln ala lys ser val ala glu val ala lys ile


1082                                                1112
TGC TAT GAA ATA GGA AAC CGC CAT GCG GCT TTA TAC TAT GGC GAG TCT CAA TTT GAA TTT
cys tyr glu ile gly asn arg his ala ala leu tyr tyr gly glu ser gln phe glu phe


1142                                                1172
AAA ACA CCA TTT GAA AAG CCC ACG CTA GCG TTA CTA GAA AAG CTA GGG GTT CAA AAT CGT
lys thr pro phe glu lys pro thr leu ala leu leu glu lys leu gly val gln asn arg


1202                                                1232
GTT TTA AGT TCA AAA TTG GAT TCC AAA GAA CGC TTA ACC GTG AGC ATG CCC CAT AGT GAG
val leu ser ser lys leu asp ser lys glu arg leu thr val ser met pro his ser glu
```

EP 0 610 322 B1

FIGURE 4 (suite)

1262                                          1292                                    SD
CCT AAT TTT AAG GTC TCA CTG GCG AGC GAT TTT AAA GTG GTC ATG AAA TAG AAA AAC AA
pro asn phe lys val ser leu ala ser asp phe lys val val met lys AMB

1321                                          1351
CAA ATG GAT AAA GGA AAA AGC GTG AAA AGC ATT GAA AAA AGC GTG GGT ATG CTC CCA AAA
F   Met asp lys gly lys ser val lys ser ile glu lys ser val gly met leu pro lys

1381                                          1411
ACT CCA AAG ACA GAC AGC AAT GCT CAT GTG GAT AAT GAA TTT CTG ATT CTG CAA GTC AAT
thr pro lys thr asp ser asn ala his val asp asn glu phe leu ile leu gln val asn

1441                                          1471
GAT GCG GTG TTC CCC ATT GGA TCT TAC ACG CAT TCT TTT GGG CTT TTG GCT AGA AAC TTA
asp ala val phe pro ile gly ser tyr thr his ser phe gly leu leu ala arg asn leu

1501                                          1531
CAT CCA GCA AAA AAG GTT ACT AAT AAA GAA AGC GCT TTA AAA TAT TTA AAA GCC AAT CTC
his pro ala lys lys val thr asn lys glu ser ala leu lys tyr leu lys ala asn leu

1561                                          1591
TCT AGC CAG TTC CTT TAC ACG GAA ATG CTG AGC TTG AAA CTC ACC TAT GAA AGC GCT CTC
ser ser gln phe leu tyr thr glu met leu ser leu lys leu thr tyr glu ser ala leu

1621                                          1651
CAA CAA GAT TTA AAA AGG ATC TTA GGG GTT GAA GAA ATC ATT ACG CTA TCC ACA AGC CCC
gln gln asp leu lys arg ile leu gly val glu glu ile ile thr leu ser thr ser pro

FIGURE 4 (Suite)

1681                                              1711
ATG GAA TTG CGA TTA GCC AAT CAA AAG CTA GGC AAT CGT TTC ATT AAA ACC TTA CAA GCC
met glu leu arg leu ala asn gln lys leu gly asn arg phe ile lys thr leu gln ala

1741                                              1771
ATG AAC GAA TTA GAC ATT GGC GCA TTT TTT AAC GCT TAC GCT CAA CAA ACC GAA GAC CCC
met asn glu leu asp ile gly ala phe phe asn ala tyr ala gln gln thr glu asp pro

1801                                                  1831
ACC CAT GCC ACT AGC TAT GGC GTT TTT GCG GCG AGT TTG GGG ATT GAA TTG AAA AAG GCT
thr his ala thr ser tyr gly val phe ala ala ser leu gly ile glu leu lys lys ala

1861                                              1891
TTA AGG CAT TAT CTT TAT GCA CAA ACT TCT AAC ATG GTA ATT AAC TGC GTT AAA AGC GTC
leu arg his tyr leu tyr ala gln thr ser asn met val ile asn cys val lys ser val

1921                                              1951
CCA CTA TCT CAA AAC GAT GGG CAA AAA ATC TTA TTG AGC TTG CAA AGC CCT TTT AAC CAG
pro leu ser gln asn asp gly gln lys ile leu leu ser leu gln ser pro phe asn gln

1981                                              2011
CTC ATA GAA AAA ACC CTA GAA CTA GAC GAA AGC CAC TTG TGC GCG GCA AGC GTT CAA AAC
leu ile glu lys thr leu glu leu asp glu ser his leu cys ala ala ser val gln asn

2041                                              2071
GAC ATT AAG GCG ATG CAG CAT GAG AGT TTA TAC TCG CGC CTT TAT ATG TCT TGA ATT TTA
asp ile lys ala met gln his glu ser leu tyr ser arg leu tyr met ser OPA

FIGURE 4 (Suite)

2102           S D                       2132

TCT CAA ATT GAA AGG AAT TTT ATG GTA AAA ATT GGA GTT TGT GGT CCT GTA GGA AGC GGT
   ureG                     Met val lys ile gly val cys gly pro val gly ser gly

2162                            2192

AAA ACC GCC TTG ATT GAA GCT TTA ACG CGC CAC ATG TCA AAA GAT TAT GAC ATG GCG GTC
lys thr ala leu ile glu ala leu thr arg his met ser lys asp tyr asp met ala val


2222                            2252

ATC ACT AAT GAT ATT TAC ACG AAA GAA GAC GCA GAA TTT ATG TGT AAA AAT TCG GTG ATG
ile thr asn asp ile tyr thr lys glu asp ala glu phe met cys lys asn ser val met


2282                            2312

CCA CGA GAG AGG ATC ATT GGC GTA GAA ACA GGA GGC TGT CCG CAC ACG GCT ATT AGA GAA
pro arg glu arg ile ile gly val glu thr gly gly cys pro his thr ala ile arg glu


2342                            2372

GAC GCT TCT ATG AAT TTA GAA GCC GTA GAA GAA ATG CAT GGC CGT TTC CCT AAT TTG GAA
asp ala ser met asn leu glu ala val glu glu met his gly arg phe pro asn leu glu


2402                            2432

TTG CTT TTG ATT GAA AGC GGA GGC AGT AAC CTT TCA GCG ACT TTC AAC CCA GAG CTA GCG
leu leu leu ile glu ser gly gly ser asn leu ser ala thr phe asn pro glu leu ala


2462                            2492

GAC TTT ACG ATC TTT GTG ATT GAT GTG GCT GAG GGC GAT AAA ATC CCC AGA AAA GGC GGG
asp phe thr ile phe val ile asp val ala glu gly asp lys ile pro arg lys gly gly

FIGURE 4 (suite)

2522                                                    2552
CCA GGA ATC ACG CGT TCA GAC TTG CTT GTC ATC AAT AAG ATT GAT TTA GCC CCC TAT GTG
pro gly ile thr arg ser asp leu leu val ile asn lys ile asp leu ala pro tyr val

2582                                                    2612
GGA GCC GAC TTG AAA GTC ATG GAA AGG GAT TCT AAA AAA ATC GCG GCG AAA AGC CCT TTA
gly ala asp leu lys val met glu arg asp ser lys lys ile ala ala lys ser pro leu

2642                                                    2672
TTT TTA CCG AAT ATC CGC GCT AAA GAA GGT TTA GAC GAT GTG ATC GCT TGG ATC AAG CGC
phe leu pro asn ile arg ala lys glu gly leu asp asp val ile ala trp ile lys arg

2702
AAC GCT TTA TTG GAA GAT TGA TGA ACA CTT
asn ala leu leu glu asp OPA

2701                S D                    2731
CAA CGC TTT ATT GGA AGA TTG ATG AAC ACT TAC GCT CAA GAA TCC AAG CTC AGG TTA AAA
        ureH                      Met asn thr tyr ala gln glu ser lys leu arg leu lys

2761                                                    2791
ACC AAA ATA GGG GCT GAC GGG CGG TGC GTG ATT GAA GAC AAT TTT TTC ACG CCC CCC TTT
thr lys ile gly ala asp gly arg cys val ile glu asp asn phe phe thr pro pro phe

2821                                                    2851
AAG CTC ATG GCG CCC TTT TAC CCT AAA GAC GAT TTA GCG GAA ATC ATG CTT TTA GCG GTA
lys leu met ala pro phe tyr pro lys asp asp leu ala glu ile met leu leu ala val

EP 0 610 322 B1

FIGURE 4 (suite)

2881 ... 2911

```
AGC CCT GGC TTA ATG AAA GGC GAT GCA CAA GAT GTG CAA TTG AAC ATC GGT CCA AAT TGC
ser pro gly leu met lys gly asp ala gln asp val gln leu asn ile gly pro asn cys
```

2941 ... 2971

```
AAG TTA AGG ATC ACT TCG CAA TCC TTT GAA AAA ATC CAT AAC ACT GAA GAC GGG TTT GCT
lys leu arg ile thr ser gln ser phe glu lys ile his asn thr glu asp gly phe ala
```

3001 ... 3031

```
AGC AGA GAC ATG CAT ATC GTT GTG GGG GAA AAC GCT TTT TTA GAC TTC GCG CCC TTC CCG
ser arg asp met his ile val val gly glu asn ala phe leu asp phe ala pro phe pro
```

3061 ... 3091

```
TTA ATC CCC TTT GAA AAC GCG CAT TTT AAG GGC AAT ACC ACG ATT TCT TTG CGC TCT AGC
leu ile pro phe glu asn ala his phe lys gly asn thr thr ile ser leu arg ser ser
```

3121 ... 3151

```
TCC CAA TTG CTC TAT AGT GAA ATC ATT GTC GCA GGG CGA GTG GCG CGC AAT GAG TTG TTT
ser gln leu leu tyr ser glu ile ile val ala gly arg val ala arg asn glu leu phe
```

3181 ... 3211

```
AAA TTC AAC CGC TTG CAC ACC AAA ATC TCT ATT TTA CAA GAT GAG AAA CCC ATC TAT TAT
lys phe asn arg leu his thr lys ile ser ile leu gln asp glu lys pro ile tyr tyr
```

3241 ... 3271

```
GAC AAC ACG ATT TTA GAT CCC AAA ACC ACC GAC TTA AAT AAC ATG TGC ATG TTT GAT GGC
asp asn thr ile leu asp pro lys thr thr asp leu asn asn met cys met phe asp gly
```

EP 0 610 322 B1

FIGURE 4 (Suite)

3301                                                      3331
TAT ACG CAT TAT TTG AAT TTG GTG CTG GTC AAT TGC CCC ATA GAG CTG TCT GGC GTG CGA
tyr thr his tyr leu asn leu val leu val asn cys pro ile glu leu ser gly val arg


3361                                                      3391
GGA TTG ATT GAA GAG AGC GAA GGA GTG GAT GGA GCC GTG AGT GAA ATC GCT AGT TCT CAT
gly leu ile glu glu ser glu gly val asp gly ala val ser glu ile ala ser ser his


3421                                                      3451
TTA TGC CTG AAA GCT TTA GCG AAA GGC TCA GAA CCC TTG TTG CAT TTA AGA GAA AAA ATC
leu cys leu lys ala leu ala lys gly ser glu pro leu leu his leu arg glu lys ile


3481                                                      3511
GCT CGC TTT ATC ACG CAA ACG ATT ACG CCA AAG GTT TAA AAA ACA CTT TAA AAA AGA TTA
ala arg phe ile thr gln thr ile thr pro lys val OCH


3541
TAC CCT TTA GTC TTT TTT AA

FIGURE 5

FIG. 6

FIGURE 7

N6::TnKm

N6

85P

FIGURE 8

EP 0 610 322 B1

FIGURE 9

FIGURE 10

Anti-urease dilué          Antiserum humain dilué
1:1,000                         1:1,000

FIGURE 11

## FIGURE 12

FIGURE 13